# EUROPEAN PATENT SPECIFICATION

(11) **EP 3 500 668 B1**
(45) Date of publication and mention of the grant of the patent: **17.06.2020**
(21) Application number: 17790663.3
(22) Date of filing: 16.08.2017
(51) Int. Cl.: C12N 15/10

(54) **DRUG-TARGET IDENTIFICATION BY RAPID SELECTION OF DRUG RESISTANCE MUTATIONS**
IDENTIFIZIERUNG DES ZIELS EINES MEDIKAMENTES DURCH SCHNELLE SELEKTION MEDIKAMENTENRESISTENTER MUTATIONEN
IDENTIFICATION DE CIBLE THÉRAPEUTIQUE PAR SÉLECTION RAPIDE DE MUTATIONS RESPONSABLES DE RÉSISTANCE À L'AGENT THÉRAPEUTIQUE

(30) Priority: 17.08.2016 GB 201614078; 18.07.2017 GB 201711520
(43) Date of publication of application: 26.06.2019
(73) Proprietor: Katholieke Universiteit Leuven, 3000 Leuven (BE)
(72) Inventor: DAELEMANS, Dirk, 3400 Ezemaal (BE); NEGGERS, Jasper, 4824 ED Breda (NL)
(74) Representative: LC Patents
(86) International application number: PCT/BE2017/000037
(87) International publication number: WO 2018/032063

(56) References cited:
- SILVANA KONERMANN ET AL: "Genome-scale transcriptional activation by an engineered CRISPR-Cas9 complex", NATURE, vol. 517, no. 7536, 10 December 2014 (2014-12-10), pages 583-588, XP055294959, ISSN: 0028-0836, DOI: 10.1038/nature14136
- O. SHALEM ET AL: "Genome-Scale CRISPR-Cas9 Knockout Screening in Human Cells", SCIENCE, vol. 343, no. 6166, 12 December 2013 (2013-12-12), pages 84-87, XP055115506, ISSN: 0036-8075, DOI: 10.1126/science.1247005 cited in the application
- JASPER E. NEGGERS ET AL: "Identifying Drug-Target Selectivity of Small-Molecule CRM1/XPO1 Inhibitors by CRISPR/Cas9 Genome Editing", CHEMISTRY & BIOLOGY, vol. 22, no. 1, 1 January 2015 (2015-01-01), pages 107-116, XP055213510, ISSN: 1074-5521, DOI: 10.1016/j.chembiol.2014.11.015 cited in the application
- T. WANG ET AL: "Genetic Screens in Human Cells Using the CRISPR-Cas9 System", SCIENCE, vol. 343, no. 6166, 12 December 2013 (2013-12-12), pages 80-84, XP055115509, ISSN: 0036-8075, DOI: 10.1126/science.1246981 cited in the application
- CORYNN KASAP ET AL: "DrugTargetSeqR: a genomics- and CRISPR-Cas9-based method to analyze drug targets", NATURE CHEMICAL BIOLOGY, vol. 10, no. 8, 1 August 2014 (2014-08-01) , pages 626-628, XP055367794, Basingstoke ISSN: 1552-4450, DOI: 10.1038/nchembio.1551 cited in the application
- YEGOR SMURNYY ET AL: "DNA sequencing and CRISPR-Cas9 gene editing for target validation in mammalian cells", NATURE CHEMICAL BIOLOGY, vol. 10, no. 8, 1 August 2014 (2014-08-01) , pages 623-625, XP055167932, ISSN: 1552-4450, DOI: 10.1038/nchembio.1550 cited in the application
- PELLAGATTI ANDREA ET AL: "Application of genome editing technologies to the study and treatment of hematological disease", ADVANCES IN BIOLOGICAL REGULATION, ELSEVIER, AMSTERDAM, NL, vol. 60, 26 September 2015 (2015-09-26), pages 122-134, XP029394606, ISSN: 2212-4926, DOI: 10.1016/J.JBIOR.2015.09.005
- DELEIDI MICHELA ET AL: "Genome editing in pluripotent stem cells: research and therapeutic applications", BIOCHEMICAL AND BIOPHYSICAL RESEARCH COMMUNICATIONS, ELSEVIER, AMSTERDAM, NL, vol. 473, no. 3, 27 February 2016 (2016-02-27), pages 665-674, XP029528745, ISSN: 0006-291X, DOI: 10.1016/J.BBRC.2016.02.113
- JONATHAN L. SCHMID-BURGK ET AL: "CRISPaint allows modular base-specific gene tagging using a ligase-4-dependent mechanism", NATURE COMMUNICATIONS, vol. 7, 28 July 2016 (2016-07-28), page 12338, XP055430010, DOI: 10.1038/ncomms12338
- SARAH A WACKER ET AL: "Using transcriptome sequencing to identify mechanisms of drug action and resistance", NATURE CHEMICAL BIOLOGY, vol. 8, no. 3, 12 February 2012 (2012-02-12), pages 235-237, XP055429409, Basingstoke ISSN: 1552-4450, DOI: 10.1038/nchembio.779
- Jasper E Neggers ET AL: "Abstract 2442: XPO1 is selinexor prime target: validation by mutating cysteine 528 on both XPO1 alleles using CRISPR/Cas9 genome editing", Cancer Research, 1 August 2015 (2015-08-01), pages 2442-2442, XP055429403, DOI: 10.1158/1538-7445.AM2015-2442 Retrieved from the Internet: URL:https://karyopharm.com/wp-content/uplo ads/2016/03/12_Neggers_Daelemans_cys528_CR ISPR_case_AACR_2015-1.pdf

### SEQUENCE LISTING

<110> Katholieke Universiteit Leuven
<120> Drug-target identification by rapid selection of drug resistance mutations
<130> 916102-GWsv
<140> PCT(NYK)
   <141> 2017-08-16
<150> GB1711520.5
   <151> 2017-07-18
<150> GB1614078.2
   <151> 2016-08-17
<160> 65
<170> PatentIn version 3.5
<210> 1
   <211> 24
   <212> DNA
   <213> homo sapiens
<400> 1
   ggattatgtg aacagaaaag aggc 24
<210> 2
   <211> 24
   <212> DNA
   <213> homo sapiens
<400> 2
   taatttcagg atcccttggc tggt 24
<210> 3
   <211> 25
   <212> DNA
   <213> homo sapiens
<400> 3
   cagctatgga aaagtcaagc tggtc 25
<210> 4
   <211> 11
   <212> PRT
   <213> homo sapiens
<400> 4
   Gly Leu Cys Glu Gln Lys Arg Gly Lys Asp Asn
   1               5                   10
<210> 5
   <211> 11
   <212> PRT
   <213> Artificial Sequence
<220>
   <223> XP01 mutant
<400> 5
   Gly Leu Ser Val His Ile Arg Gly Lys Asp Asn
   1               5                   10
<210> 6
   <211> **11**
   <212> PRT
   <213> Artificial Sequence
<220>
   <223> XP01 mutant
<400> 6
   Gly Tyr Val Asn Arg Lys Arg Gly Lys Asp Asn
   1 5 10
<210> 7
   <211> 7
   <212> PRT
   <213> Artificial Sequence
<220>
   <223> XPO1 mutant
<400> 7
   Gly Tyr Arg Gly Lys Asp Asn
   1 5
<210> 8
   <211> 10
   <212> PRT
   <213> Artificial Sequence
<220>
   <223> XP01 mutant
<400> 8
   Gly Asp Pro Pro Val Arg Gly Lys Asp Asn
   1 5 10
<210> 9
   <211> 11
   <212> PRT
   <213> Artificial Sequence
<220>
   <223> XP01 mutant
<220>
   <221> MISC_FEATURE
   <222> (11)..(11)
   <223> Xaa is stop
<400> 9
   Gly Leu Cys Glu Gln Lys Lys Arg Gln Arg Xaa
   1 5 10
<210> 10
   <211> 11
   <212> PRT
   <213> Artificial Sequence
<220>
   <223> XPO1 mutant
<400> 10
   Gly Leu Cys Glu Gln Lys Glu Ala Lys Ile Ile
   1 5 10
<210> 11
   <211> 9
   <212> PRT
   <213> Artificial Sequence
<220>
   <223> XP01 mutant
<220>
   <221> MISC_FEATURE
   <222> (9)..(9)
   <223> Xaa is stop
<400> 11
   Gly Leu Cys Glu Gln Arg Gln Arg Xaa
   1 5
<210> 12
   <211> 8
   <212> PRT
   <213> **homo sapiens**
<400> 12
   Asp Pro Leu Ala Gly Ile Ile Pro
   1 5
<210> 13
   <211> 5
   <212> PRT
   <213> Artificial Sequence
<220>
   <223> KIF11 mutant
<400> 13
   Ala Gly Ile Ile Pro
   1 5
<210> 14
   <211> 7
   <212> PRT
   <213> Artificial Sequence
<220>
   <223> KIF11 mutant
<400> 14
   Asp Pro Ala Gly Ile Ile Pro
   1 5
<210> 15
   <211> 9
   <212> PRT
   <213> Artificial Sequence
<220>
   <223> KIF11 mutant
<400> 15
   Asp Pro Phe Leu Ala Gly Ile Ile Pro
   1 5
<210> 16
   <211> 5
   <212> PRT
   <213> Artificial Sequence
<220>
   <223> KIF11 mutant
<400> 16
   Asp Gly Ile Ile Pro
   1 5
<210> 17
   <211> 6
   <212> PRT
   <213> Artificial Sequence
<220>
   <223> KIF11 mutant
<400> 17
   Asp Pro Gly Ile Ile Pro
   1 5
<210> 18
   <211> 7
   <212> PRT
   <213> Artificial Sequence
<220>
   <223> KIF11 mutant
<400> 18
   Asp Leu Ala Gly Ile Ile Pro
   1 5
<210> 19
   <211> 7
   <212> PRT
   <213> Artificial Sequence
<220>
   <223> KIF11 mutant
<400> 19
   Asp Pro Ala Gly Ile Ile Pro
   1 5
<210> 20
   <211> 8
   <212> PRT
   <213> Artificial Sequence
<220>
   <223> KIF11 mutant
<400> 20
   Asp Pro Phe Gly Trp Tyr Asn Ser
   1 5
<210> 21
   <211> 6
   <212> PRT
   <213> Artificial Sequence
<220>
   <223> KIF11 mutant
<400> 21
   Asp Pro Trp Tyr Asn Ser
   1 5
<210> 22
   <211> 12
   <212> PRT
   <213> Artificial Sequence
<220>
   <223> KIF11 mutant
<220>
   <221> MISC_FEATURE
   <222> (5)..(5)
   <223> Xaa is stop
<400> 22
   Asp Pro Leu Leu Xaa Phe Gln Ala Gly Ile Ile Pro
   1 5 10
<210> 23
   <211> 8
   <212> PRT
   <213> homo sapiens
<400> 23
   Ser Tyr Gly Lys Val Lys Leu Val
   1 5
<210> 24
   <211> 9
   <212> PRT
   <213> Artificial Sequence
<220>
   <223> ERCC3 mutant
<400> 24
   Ser Tyr Gly Lys Val Leu Lys Leu Val
   1 5
<210> 25
   <211> 9
   <212> PRT
   <213> Artificial Sequence
<220>
   <223> ERCC3 mutant
<220>
   <221> MISC_FEATURE
   <222> (6)..(6)
   **<223> Xaa is stop**
**<400> 25**
   Ser Tyr Gly Lys Val Xaa Gln Leu Val
   1 5
<210> 26
   <211> 6
   <212> PRT
   <213> Artificial Sequence
<220>
   <223> ERCC3 mutant
<400> 26
   Ser Tyr Gly Lys Leu Val
   1 5
<210> 27
   <211> 9
   <212> PRT
   <213> Artificial Sequence
<220>
   <223> ERCC3 mutant
<220>
   <221> MISC_FEATURE
   <222> (6)..(6)
   <223> Xaa is stop
<400> 27
   Ser Tyr Gly Lys Val Xaa Lys Leu Val
   1 5
<210> 28
   <211> 10
   <212> PRT
   <213> Artificial Sequence
<220>
   <223> ERCC3 mutant
<400> 28
   Ser Tyr Gly Lys Ala Met Glu Lys Leu Val
   1 5 10
<210> 29
   <211> 5
   <212> PRT
   <213> Artificial Sequence
<220>
   <223> ERCC3 mutant
<400> 29
   Ser Tyr Gly Lys Val
   1 5
<210> 30
   <211> 9
   <212> PRT
   <213> Artificial Sequence
<220>
   <223> ERCC3 mutant
<400> 30
   Ser Tyr Gly Lys Val Val Lys Leu Val
   1 5
<210> 31
   <211> 8
   <212> PRT
   <213> Artificial Sequence
<220>
   <223> ERCC3 mutant
<400> 31
   Ser Tyr Gly Asn Val Lys Leu Val
   1 5
<210> 32
   <211> 10
   <212> PRT
   <213> Artificial Sequence
<220>
   <223> ERCC3 mutant
<220>
   <221> MISC_FEATURE
   <222> (7)..(7)
   <223> Xaa is stop
<400> 32
   Ser Tyr Gly Lys Val Thr Xaa Gln Leu Val
   1 5 10
<210> 33
   <211> 20
   <212> DNA
   <213> homo sapiens
<400> 33
   gtaagcaccc gctgtagccc 20
<210> 34
   <211> 20
   <212> DNA
   <213> homo sapiens
<400> 34
   tgggctcggt gctgccctac 20
<210> 35
   <211> 20
   <212> DNA
   <213> homo sapiens
<400> 35
   caccatggct gggggcgcag 20
<210> 36
   <211> 20
   <212> DNA
   <213> homo sapiens
<400> 36
   cggcatgccg cagggcagtg 20
<210> 37
   <211> 20
   <212> DNA
   <213> homo sapiens
<400> 37
   cagtactcag cctggcaagg 20
<210> 38
   <211> 36
   <212> DNA
   <213> homo sapiens
<400> 38
   ttatttacag gatctattag gattatgtga acagaa 36
<210> 39
   <211> 36
   <212> DNA
   <213> homo sapiens
<400> 39
   taatttcagg atcccttggc tggtataatt ccacgt 36
<210> 40
   <211> 36
   <212> DNA
   <213> homo sapiens
<400> 40
   tatttgcagt tgtgtactgt cagctatgga aaagtc 36
<210> 41
   <211> 11
   <212> PRT
   <213> homo sapiens
<400> 41
   Asp Leu Leu Gly Leu Cys Glu Gln Lys Arg Gly
   1 5 10
<210> 42
   <211> 9
   <212> PRT
   <213> Artificial Sequence
<220>
   <223> XP01 mutant
<400> **42**
   Asp Leu Leu Gly Leu Gln Lys Arg Gly
   1 5
<210> 43
   <211> 11
   <212> PRT
   <213> Artificial Sequence
<220>
   <223> XP01 mutant
<400> 43
   Asp Leu Leu Gly Cys Asn Glu Gln Lys Arg Gly
   1 5 10
<210> 44
   <211> 10
   <212> PRT
   <213> Artificial Sequence
<220>
   <223> XP01 mutant
<400> 44
   Asp Leu Leu Asp Tyr Val Gln Lys Arg Gly
   1 5 10
<210> 45
   <211> 11
   <212> PRT
   <213> Artificial Sequence
<220>
   <223> XP01 mutant
<400> 45
   Asp Leu Leu Glu Lys Ile Tyr Gln Lys Arg Gly
   1 5 10
<210> 46
   <211> 9
   <212> **PRT**
   <213> Artificial Sequence
<220>
   <223> XP01 mutant
<400> 46
   Asp Leu Leu Gly Leu Gln Lys Arg Gly
   1 5
<210> 47
   <211> 10
   <212> PRT
   <213> Artificial Sequence
<220>
   <223> XPO1 mutant
<220>
   <221> MISC_FEATURE
   <222> (5)..(5)
   <223> Xaa is stop
<400> 47
   Asp Leu Leu Val Xaa Thr Glu Lys Arg Gln
   1 5 10
<210> 48
   <211> 8
   <212> PRT
   <213> homo sapiens
<400> 48
   Asp Pro Leu Ala Gly Ile Ile Pro
   1 5
<210> 49
   <211> 8
   <212> PRT
   <213> Artificial Sequence
<220>
   <223> KIF11 mutant
<400> 49
   Asp Pro Leu Val Gly Ile Ile Pro
   1 5
<210> 50
   <211> 9
   <212> PRT
   <213> homo sapiens
<400> 50
   Leu Cys Thr Val Ser Tyr Gly Lys Val
   1 5
<210> 51
   <211> 8
   <212> PRT
   <213> Artificial Sequence
<220>
   <223> ERCC3 mutant
<400> 51
   Leu Cys Thr Val Asn Gly Lys Val
   1 5
<210> 52
   <211> 10
   <212> PRT
   <213> Artificial Sequence
<220>
   <223> ERCC3 mutant
<400> 52
   Leu Cys Thr Val Ser Tyr Asp Gly Lys Val
   1 5 10
<210> 53
   <211> 9
   <212> PRT
   <213> Artificial Sequence
<220>
   <223> ERCC3 mutant
<400> 53
   Leu Cys Thr Val Ser Tyr Glu Lys Val
   1 5
<210> 54
   <211> 8
   <212> PRT
   <213> Artificial Sequence
<220>
   <223> ERCC3 mutant
<400> 54
   Leu Cys Thr Val Ile Gly Lys Val
   1               5
<210> 55
   <211> 7
   <212> PRT
   <213> Artificial Sequence
<220>
   <223> ERCC3 mutant
<400> 55
   Leu Cys Thr Val Ser Lys Ser
   1               5
<210> 56
   <211> 6
   <212> PRT
   <213> Artificial Sequence
<220>
   <223> ERCC3 mutant
<400> 56
   Leu Cys Thr Val Lys Ser
   1               5
<210> 57
   <211> 8
   <212> PRT
   <213> Artificial Sequence
<220>
   <223> ERCC3 mutant
<400> 57
   Leu Cys Thr Val Ser Tyr Glu Lys
   1               5
<210> 58
   <211> 9
   <212> PRT
   <213> homo sapiens
<400> 58
   Asp Leu Leu Gly Leu Cys Glu Gln Lys
   1 5
<210> 59
   <211> 7
   <212> PRT
   <213> Artificial Sequence
<220>
   <223> XPO1 mutant
<400> 59
   Asp Leu Leu Gly Leu Gln Lys
   1 5
<210> 60
   <211> 9
   <212> PRT
   <213> Artificial Sequence
<220>
   <223> XP01 mutant
<400> 60
   Asp Leu Leu Glu Asp Ile Leu Gln Lys
   1 5
<210> 61
   <211> 8
   <212> PRT
   <213> homo sapiens
<400> 61
   Gly Leu Cys Glu Gln Lys Arg Gly
   1 5
<210> 62
   <211> 5
   <212> PRT
   <213> homo sapiens
<400> 62
   Asp Pro Leu Ala Gly
   1 5
<210> 63
   <211> 8
   <212> PRT
   <213> homo sapiens
<400> 63
   Asp Leu Leu Gly Leu Cys Glu Gln
   1               5
<210> 64
   <211> 9
   <212> PRT
   <213> homo sapiens
<400> 64
   Asp Pro Leu Ala Gly Ile Ile Pro Arg
   1               5
<210> 65
   <211> 19
   <212> DNA
   <213> homo sapiens
<400> 65
   taatttctac tcttgtaga 19

## Description

### FIELD OF THE INVENTION

The present invention relates to methods used in functional genomics that focus on gene function in a cell. The invention also relates to mutagenizing genes and generation of functional genetic mutants. The current invention also relates to methods for stimulus/drug-target identification. In addition, the invention relates to the generation of cell lines showing a functional phenotype, most notably stimulus/drug-resistant cell lines. The current invention further relates to methods for identification of mutations conferring this phenotype. The current invention further relates to said methods and provides for rapid selection methods to identity targets and to identify stimulus/drug-target interactions and to identify mutations conferring stimulus/drug-resistance, more specifically said methods comprise the use of CRISPR/Cas systems, components thereof or the like.

### BACKGROUND OF THE INVENTION

Identifying the cellular target of a chemical hit with valuable activity is a crucial step in drug discovery and development. However, unraveling the molecular target of small molecules remains a challenging, laborious and complex process. Although target deconvolution methods have successfully been applied, they often reveal more than one plausible candidate target protein and carry the risk of identifying interactions that are not related to the compound's activity. The gold standard proof of a small molecule's direct target is the discovery of functional mutations that confer resistance in a human cellular context. Therefore, genetic screens are very powerful tools for drug mechanism of action studies. However, current screens either are not well suited to identify essential genes in a drugs mechanism of action or require whole exome sequencing combined with complex bio-informatics to deconvolute the relevant drug resistance conferring mutations. For example, loss-of-function approaches have been applied to obtain drug resistance (Shalem et al. 2014¹¹, Wang et al. 2014¹²), but these innately lack the ability to comprehensively detect gain-of-fundion mutations in essential proteins. Indeed, inactivation of the expression of essential genes would cause a lethal phenotype by themselves precluding selection and identification of these essential genes. Because many cancer drugs target essential proteins, there is a need for an accessible method that can easily generate resistance to drugs targeting these essential genes. While classical step-wise drug resistance selection in cancer cells is laborious and often results in off-target multi-drug resistance, genetic screening using chemical induction of single-nucleotide variants in haploid cells was recently reported. However, this chemical mutagenesis approach requires haploid cells and is therefore restricted to compounds active in these cell types. Another bottleneck of random mutagenesis coupled to drug resistance selection is the identification of the relevant mutations that confer resistance. Due to the human's large genome size in addition to the heterogeneity of the cell line, this identification process requires whole transcriptome sequencing coupled to extensive bioinformatic analysis and validation (Wacker et al. 2012, Kasap et al. 2014, Smurnyy et al. 2014)¹⁻⁴. As such, the field still needs and would greatly benefit from a methodology that can speed up the drug resistance selection process and simplify subsequent identification of the relevant drug resistance mutations.

### SUMMARY OF THE INVENTION

The invention is defined by the appended claims. The present Inventors have found methods to rapidly generate functional mutations in proteins that confer a phenotype, most notably resistance against a stimulus or a drug, using genome-editing technology, such as the RNA-guided CRISPR/Cas system. More specifically, said methods can be applied for the identification of a target for a certain drug or stimulus. The methods of the present invention comprise the use of specific guide RNA's, without using another template substrate to induce homology-directed repair (HDR). Therefore, in the methods of the present invention, mutations are generated by non-homologous end-joining (NHEJ). Said NHEJ can facilitate in-frame mutagenesis and the efficiency of said in-frame mutations, indels or base substitutions is a specific feature of the current methods of the present invention and differs from out-of-frame mutations causing protein inactivation/loss-of-function.

Furthermore, said methods can be used to rapidly generate functional protein variants in organisms such as plants, yeast, bacteria, viruses, and mammalian cells; more specifically they can be applied to identify the specific drug-target interaction site and can be used to rapidly generate mutations that confer resistance to a stimulus, a bioactive molecule or a pathogen; examples of said stimulus are drugs, more specifically anti-cancer drugs, other examples of said stimulus are pathogens such as viruses, bacteria, kinetoplastids and the like. The current state of the art methods for the generation of drug/stimulus resistance, e.g. to generate stimulus/resistant cell lines, is still a cumbersome task which takes several weeks or months. With the present invention, new methods are designed in which said methods of the present invention are performed in days/weeks. The present invention provide method steps (i) to (iv) which each can be performed in 1 day to about 1 or 2 weeks. The complete method therefore can take about 6 days or about 1 or 2 weeks to about 4 or 5 weeks. The sequencing steps (a) and (b) of the present methods of the invention can take about 1 day or a few days up to about 1 or 2 weeks, depending on the sequencing technology that is used. Thus, the overall methods for generating resistant cell lines can be performed in about 1 to 3 weeks and the subsequent sequencing steps for the subsequent identification of the mutations can be performed in about 1 or more additional days or about 1 or more weeks, depending on the sequencing technology used.

The summary above is to be considered as a brief and general overview of some of the embodiments disclosed herein, is provided solely for the benefit and convenience of the reader, and is not intended to limit in any manner the scope encompassed by the appended claims.
Unless defined otherwise, all technical and scientific terms used herein have the same meaning as commonly understood by one of ordinary skill in the art in the field of the invention. Any methods and materials similar or equivalent to those described herein can also be used in the practice or the present invention, but the preferred methods and products are described herein.

### Definitions

As used herein, the singular forms "a", "an", and "the" include both singular and plural referents unless the context clearly dictates otherwise.
The terms "comprising", "comprises" and "comprised of as used herein are synonymous with "including", "includes" or "containing", "contains", and are inclusive or open-ended and do not exclude additional, non-recited members, elements or method steps. The terms "comprising", "comprises" and "comprised or when referring to recited components, elements or method steps also include embodiments which "consist of said recited components, elements or method steps.
Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The term "about" as used heroin when referring to a measurable value such as a parameter, an amount, a temporal duration, and the like, is meant to encompass variations of +/-20% or +/-10% or less, preferably +/-5% or less, more preferably +/-1% or less, and still more preferably +/-0.1% or less of and from the specified value, insofar such variations are appropriate to perform in the disclosed invention. As an example, in case the term about is used in combination with a certain amount of days, it includes said specific amount of days plus or minus 1 day, eg. about 6 days include any amount of days between 5 and 7. It is to be understood that the value to which the modifier "about" refers is itself also specifically, and preferably, disclosed.

The term "DNA-repair enzyme" as used herein refers to an enzyme or protein present in prokaryotic or eukaryotic organisms that assist or carry out the synthesis or repair of DNA. Examples of such DNA-iepair enzymes include, but are not limited to the mouse, rat or human encoded proteins:
- proliferating cell nuclear antigen (PCNA)
- The MRN complex subunits (MRE11, RAD50, NBS1/NBN)
- DNA-dependent protein kinase catalytic subunit (PRKDC/XRCC7)
- Ku70, Ku80 and XRCC4-like factor (XRCC6, XRCC5, NHEJ1/XLF)
- DNA ligase I, III, and IV (LIG1, LIG3, LIG4)
- X-ray repair cross-complementing 1 and 4 (XRCC1, XRCC4)
- Breast cancer 1 and 2 (BRCA1, BRCA2)
- Flap endonuclease 1 (FEN) and Poly [ADP-ribose] polymerase 1 (PARP1)
- family A polymerases including:
   ∘ REV1
   ∘ Polymerase gamma (Pol γ/POLG)
   ∘ Polymerase theta (Pol δ/MPOLQ)
   ∘ Polymerase nu (Pol v/POLN)
- family B DNA polymerases including:
   ∘ Polymerase alpha (Pol α) subunits (POLA1, POLA2, PRIM1, PRIM2)
   ∘ Polymerase delta (Pol δ) subunits (POLD1, POLD2. POLD3, POLD4)
   ∘ Polymerase epsilon (Pol ε) subunits (POLE, POLE2, POLE3 POLE4)
   ∘ Polymerase zeta (Pol ζ) subunits (REV3L, REV7)
- family X DNA polymerases including:
   ∘ Polymerase beta (Pol β/POLB)
   ∘ Polymerase lamda (Pol λ/POLL)
   ∘ Polymerase mu (Pol µ/POLM)
   ∘ Polymerase sigma (Pol σ)
   ∘ Terminal Deoxynucleotidyl Transferase (TdT/DNTT)
- family Y DNA polymerases including:
   ∘ Polymerase kappa (Pol κ/POLK)
   ∘ Polymerase eta (Pol η/POLH)
   ∘ Polymerase iota (Pol ι/POLI)
- Or any protein that is at least 80, 90. 95, or 99% homologous to the natural DNA-repair enzymes, such as the proteins described hereabove.

The recitation of numerical ranges by endpoints includes all numbers and fractions subsumed within the respective ranges, as well as the recited endpoints.

The term "fused" or "fusion" as used herein to any way to recruit/localize one protein domain or complex to another protein domain or complex (subject) in close proximity. These fusion/fused ways comprise the direct fusion of one protein domain or complex to the other by covalent linking of the molecular entities, wherein the covalent link optionally comprises a peptide-linker. Fusion further comprises a transient localization or recruitment by using affinity based concepts such as antibody-antigen interactions, RNA or DNA binding protein domains In combination with DNA or RNA, dimerization or polymerization domains such as for example SH2, SH3, PDZ and other domains, chemical crosslinking and chemical-induced dimerization or light-induced dimerization, as well as other methods, all well known to the person skilled in the art. "Recruitment" or "recruits" as used herein also comprises the recruitment of DNA repair enzymes via fusion of said DNA repair enzymes (or mutants thereof) to the guide RNAs of the present invention. Examples of such fusions comprise the use of the bacteriophage RNA binding proteins MS2, R17, λ N, Qβ, PP7 or any variant or mutant thereof, the Pumilio RNA binding proteins and mutants and variants thereof and other RNA binding proteins such as the viral HIV-1 Rev protein.

The term "tiling" or "tiling sgRNA library' refers to a library containing all possible sgRNA or guide RNAs targeting the coding sequences of a gene. For example, a tiling sgRNA library targeting gene X is a library containing all possible sgRNAs that target the exonic sequences of that gene X.

### DESCRIPTION

One aspect of the present invention relates to a method for generating a stimulus resistant cell line. Said method comprises the following steps:
(i) transduce a cell line, stably expressing an RNA-guided endonuclease of the clustered regularly interspaced short palindromic repeats (CRISPR) system wherein said endonuclease generates mutations my non-homologous end-joining (NHEJ), with a vector library comprising guide RNAs targeting exonic sequences, and targeting intronic sequences within 30 base pairs of an intron-exon boundary, of at least one candidate target gene or the whole exome of the organism the stimulus is targeting, wherein the method does not comprise the use of a homology-directed repair (HDR) template or substrate;
(ii) select for transduced cells at the end of step (i);
(iii) treat selected cells at the end of step (ii) with the stimulus;
(iv) grow the stimulus resistant colonies that are formed at the end of step (iii):
(v) identify or sequence the guide RNA sequence(s) present in the resistant colonies generated in (iv);
(vi) sequence the genomic region around the target sequence of the identified guide RNA(s) to identify the genetic mutations that confer cellular resistance to the stimulus; and
(vii) select those colonies wherein the mutations consist of in-frame insertions and/or in-frame deletions and/or in-frame indels and/or point mutations, and excluding mutations that introduce a premature stop codon that leads to loss-of-function, of the identified target sequence of step (vi).

Another aspect of the present invention relates to a method for generating a mutant cell line. Said method comprises the following steps:
(i) transduce a cell line, stably expressing an RNA-guided endonuclease of the clustered regularly interspaced short palindromic repeats (CRISPR) system wherein said endonuclease generates mutations by non-homologous end-joining (NHEJ), with a vector library comprising guide RNAs targeting exonic sequences and targeting intronic sequences within 30 base pairs of an intron-exon boundary, in at least one candidate target gene or in the whole exome of the organism the stimulus is targeting, wherein the method does not comprise the use of homology-directed repair (HDR) template or substrate;
(ii) select for the transduced cells at the end of step (i);
(iii) select the cells at the end of step (ii) with a certain phenotype;
(iv) grow the selected colonies at the end of step (iii);
(v) identify or sequence the guide RNA sequence(s) present in the selected colonies generated in (iv);
(vi) sequence the genomic region around the target sequence of the identified guide RNA(s) to identify the mutations that cause said certain phenotype; and
(vii) select those colonies wherein the mutations are in frame insertions and/ or in frame deletions and/or point mutations of the identified target sequence of step (vi).

Furthermore, the methods of the present invention can be used to rapidly generate functional protein variants in organisms such as plants. yeast, bacteriae, viruses, and (mammalian) cells; more specifically they can be applied to identity the specific drug-target interaction site and can be used to rapidly generate mutations that confer resistance to a stimulus, a bioactive molecule or a pathogen for that organism.

One embodiment of the present invention relates to the methods of the present invention, wherein said methods for generating a stimulus resistant cell line are methods for generating in-frame gain-of-function mutations in proteins in cells. More specifically said methods are not designed for generating loss-of-function cell lines. Said methods are specifically designed to avoid loss-of-function cell lines and said methods can be used to generate mutant cell lines or stimulus resistant cell lines for essential genes, amongst other uses. In specific embodiments of the present invention, said gain-of-function or mutant cell line is a cell line which comprise a point mutation, an in-frame insertion or in frame deletion, which does not destroy or knock out the complete gene/function, but confers rather a specific functional mutation or a gain-of-function mutation, which destroys only a specific functional domain of said gene, leaving the rest of the gene and functions of the corresponding protein intact. The methods of the present Invention are specifically designed to select said gain-of-function or cell lines with mutated essential genes.
One embodiment of the present invention relates to the methods of the present invention, wherein the RNA-guided endonuclease is CRISPR/Cas9 or Cpf1 or any mutant thereof, such as Cas9-D10A. Cas9-H840A. Cas9-VQR, Cas9-EQR. Cas9-VRER, AsCpf1-S542R/K607R. AsCpf1-S542R/K548V/N552R, LbCpf1-G532R/K595R, LbCPf1-G532R/K538V/Y542R or any fusion thereof of any combination of a mutant and fusion thereof, such as dCas9 fused to a mutation inducing enzyme, or complex such as for example human AID, APOBEC and similar proteins. Examples of said RNA-guided endonuclease include but are not limited to: SpCas9-D10A, SpCas9-H840A, SpCas9-VQR, SpCas9-EQR, SpCas9-VRER, AsCpf1-S542R/K607R, AsCpf1-S542R/K548V/N552R, LbCpf1-G532R/K595R, LbCPf1-G532R/K538V/Y542R. Said RNA-guided endonucleases can originate from several species such as SpCas9, NmeCas9, SaCas9 etc.
One embodiment of the present invention relates to the methods of the present invention, wherein said methods do not comprise the use of a homology-directed repair (HDR) template or substrate, but rely on NHEJ to generate open-ended functional mutations.
One embodiment of the present invention relates to the methods of the present invention, wherein the vector library is a collection of tiling guide RNAs, Another embodiment of the present invention relates to the methods of the present invention, wherein the vector library is a collection of guide RNAs targeting exonic or coding sequences and intronic sequences within 30 base pairs of an intron-exon boundary.
One embodiment of the present invention relates to the methods of the present invention, wherein the vector library is a collection of tiling guide RNAs targeting only coding sequences. One embodiment of the present invention relates to the methods of the present invention, wherein the guide RNAs target sequences coding for functional domains of said at least one candidate target gene.
One embodiment of the present invention relates to the methods of the present invention, wherein the selection step (ii) and/or step (iii) is based on the selection of surviving clones or on another selectable or enrichable phenotype.
One embodiment of the present invention relates to the methods of the present invention, wherein the stimulus is a bioactive molecule with anticancer activity.
One embodiment of the present invention relates to the methods of the present invention, wherein the stimulus is a bioactive molecule inducing a selectable or enrichable phenotype.
One embodiment of the present invention relates to the methods of the present invention, wherein the stimulus is a drug, a pathogen, a virus or a bacterium. In a specific embodiment of the present invention, said stimulus is a drug and in a more specific embodiment, said drug is a cancer-drug.
One embodiment of the present invention relates to the methods of the present invention, wherein the vector library is a lentiviral vector library.
One embodiment of the present invention relates to the methods of the present invention, wherein the vector library comprises all possible guide RNAs present in the coding sequence of at least one or part of one candidate target gene. In another embodiment said vector library all possible guide RNAs present in the whole coding exome of the organism. In a specific embodiment, said organism is the organism the stimulus is targeting.
One embodiment of the present invention relates to the methods of the present invention, wherein the RNA-guided endonuclease belongs to the Clustered regularly interspaced short palindromic repeats (CRISPR) system.
One embodiment of the present invention relates to the methods of the present invention, wherein the RNA-guided endonuclease is fused with a DNA-repair enzyme, or wherein the guide RNA itself recruits (by e.g. MS2-fusion or Pumilio RNA binding-fusion proteins) a DNA-repair enzyme. including any mutant of said DNA-repair enzyme. In a specific embodiment, said DNA-repair enzyme is DNA Ligase IV, β-polymerase or θ-polymerase, including any mutant thereof.

One embodiment of the present invention relates to the methods of the present invention, wherein the vector library of step (i) comprises a selection marker and wherein in step (ii) transduced cells are selected by using that marker.
One embodiment of the present invention relates to the methods of the present invention, wherein the marker is an antibiotic resistance marker, and the transduced cells in step (ii) are selected by growing the cells in the presence of said antibiotic.
One embodiment of the present invention relates to the methods of the present invention, wherein the stimulus is lethal for the untreated, wild type cell and wherein the stimulus is lethal for all the cells in step (iii) which do not comprise a mutation conferring resistance to said stimulus at the end of step (ii).
One embodiment of the present invention relates to the methods of the present invention, wherein step (i) is about 1 day and/or step (ii) is about 5 days and/or step (iii) is about 1 to 2 weeks and/or step (iv) is about 1 to 2 weeks.
One embodiment of the present invention relates to the methods of the present invention, wherein the stimulus acts on an essential gene and the target sequence in step (vi) is part of said essential gene.

In one embodiment of the present invention, the vector library is a collection of guide RNAs targeting only exon sequences, wherein said guide RNA's are distributed over all the exons of the genes.

In one embodiment of the present invention, the RNA-guided endonuclease is fused or combined with a DNA-repair enzyme, including any mutant thereof. Both the RNA-guided endonuclease and/or the DNA-repair enzyme can be mutated, wherein said mutations are at least 80, 90, 95, or 99 % homologues to its natural or wild type counterpart.
In one embodiment, the RNA-guided endonuclease is Cas9 or Cpf1or any mutant thereof, including but not limited to Cas-D10A; Cas-H840A; Cas-VQR; Cas-EQR; Cas-VRER; such as Cas9-D10A, Cas9-H840A, Cas9-VQR, Cas9-EQR, Cas9-VRER; AsCpf-S542R/K607R; AsCpf1-S542R/548V/N552R; and LbCpf1-G532R/K595R; LbCPf1-G532R/K538V/Y542R In another embodiment, said RNA-guided endonuclease is C2c2 or any mutant thereof.

### BRIEF DESCRIPTION OF THE DRAWINGS

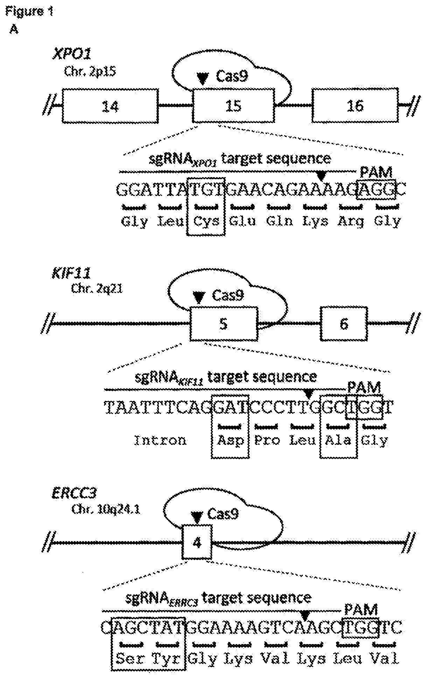

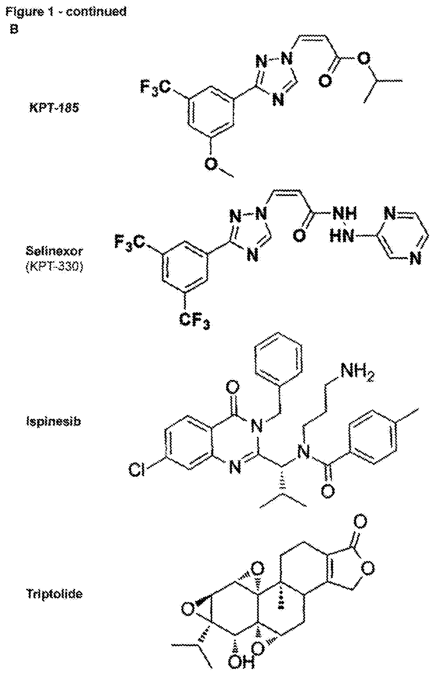

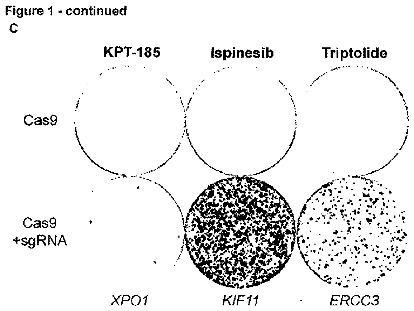

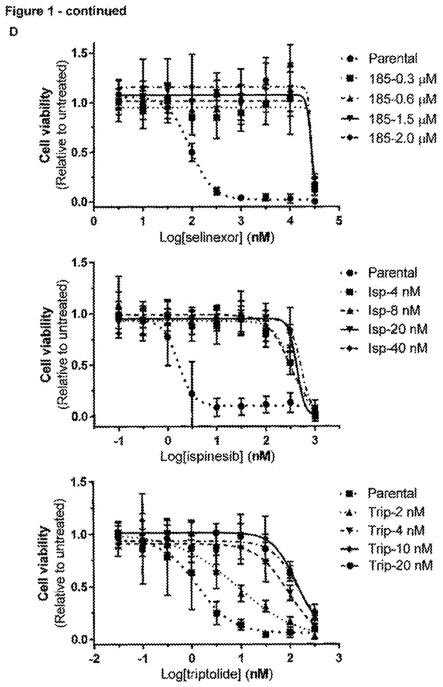

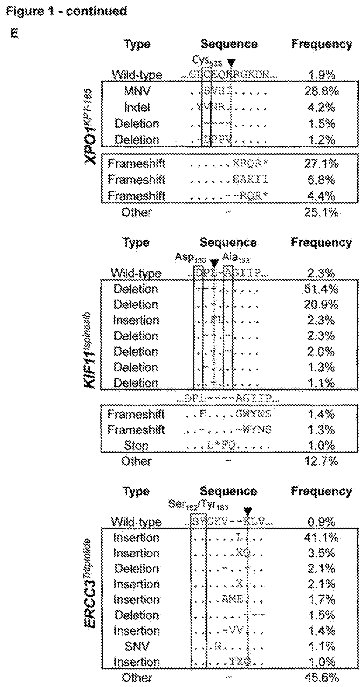

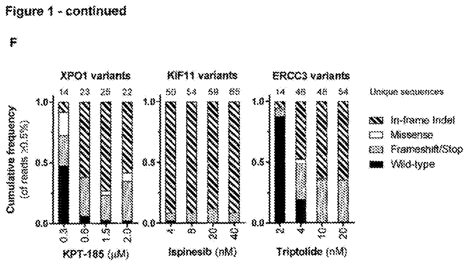

**Figure 1** **- Spontaneous genetic variation generated by CRISPR/Cas9-induced NHEJ repair facilitates rapid selection of many different drug resistant protein variants**
   a. Representation of used CRISRP/Cas9 sgRNAs targeting resistance hot spots for selinexor (*XPO1* codon C528), ispinesib (*KIF11* codons D130 and A133), and triptolide (*ERCC3* codons S162 and Y163). The Cas9 cleavage site is indicated by an arrowhead. (*Sequence IDs: 1-3,23,61,62*)
   b. Chemical structures of the antineoplastic agents KPT-185 (a preclinical analogue of selinexor with a high in vitro potency). selinexor (KPT-330), ispinesib, and triptolide.
   c. Cas9-induced DSB at resistance hot spots and subsequent NHEJ repair rapidly generates resistant colonies. Cells were transfected with either a plasmid expressing only Cas9 (top) or with two plasmids expressing Cas9 and sgRNA (bottom). The sgRNA target genes are shown below. KPT-185 (300 nM). ispinesib (4 nM), or triptolide (10 nM) were added 48 hours after transection and selection was maintained for 7-10 days before visualization.
   d. Cell viability assay showing the effect of selinexor, ispinesib or triptolide on wild-type (parental) and the mutagenized resistant cells from panel c. The experiment in c was performed with 4 different concentrations of compound for selection; KPT-185. 0.3, 0.6, 1.5 or 2 µM; ispinesib: 4. 8, 20.40 nM; triptolide: 2, 4, 10, 20 nM. Cell viability assays were performed on each of these resistant cell populations. Data points are normalized relative to DMSO treated cells and represent averages with standard deviation (N=3).
   e. Amino acid sequence variants, as determined by next generation sequencing analysis, in cells transfected with Cas9 and the respective sgRNA and selected with 0.6 pM KPT-185, 4 nM ispinesib or 10 nM triptolide. The wild-type sequence is shown for reference and resistance hot spot residues are highlighted in the vertical column. The Cas9 cleavage site is indicated by an arrowhead and a vertical dashed line. (*Sequence IDs:* 4-32)
   f. Targeted amplicon sequencing analysis by CrispRVariants of cells selected with the different concentrations of KPT-185, ispinesib or triptolide The relative abundance of alleles with a read frequency ≥0.5% is shown and categorized per sample into 4 different mutation types. Data shown in each column represents the average fractions obtained from 2 independent experiments.

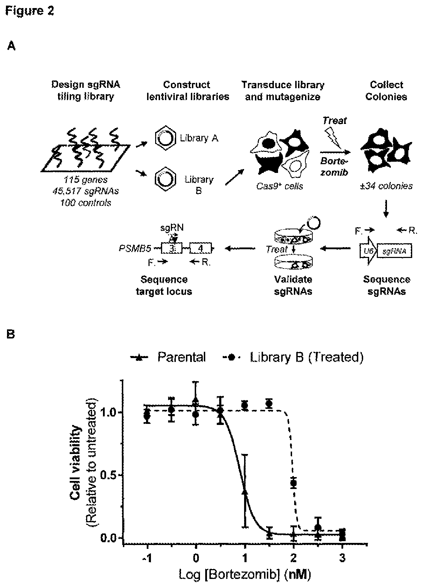

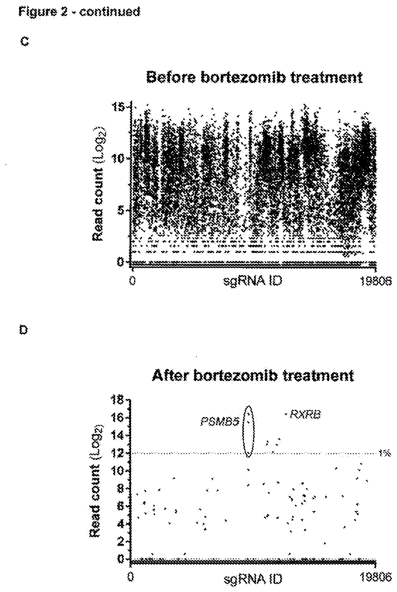

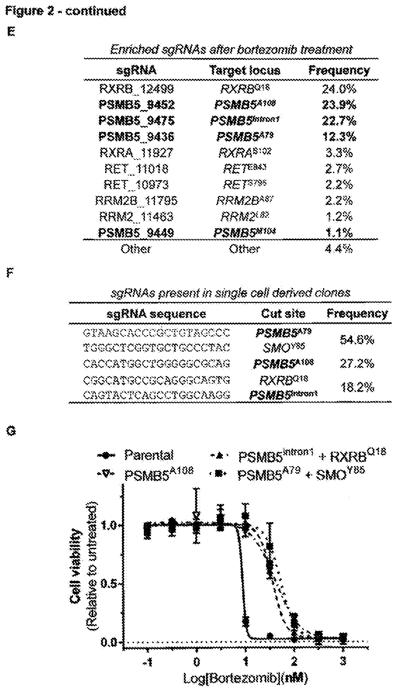

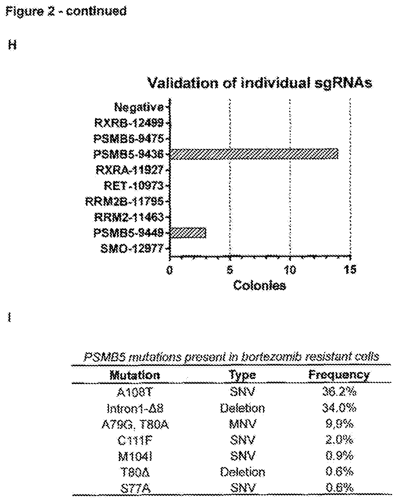

Figure 2 - Validation of the CRISPR mutagenesis scanning method using bortezomib and a large-scale "FDAtarget" CRISPR/Cas9 tiling library
   a. Overview of the workflow for the CRISPR/Cas9-based chemical target identification screen used for bortezomib. Please note that only sub-library B (64 genes, I-Z) was used for this screen.
   b. Cell viability of parental and mutagenized bortezomib resistant HAP1 cells in the presence of different concentrations of bortezomib. Data points represent means and error bars indicate standard deviation (N=3).
   c. Representation of the different sgRNAs in the transduced cell pool before treatment with 30 nM bortezomib (after puromycin selection). Each dot represents a different sgRNA.
   d. Representation of the enriched sgRNAs present in the resistant cell pool after treatment with 30 nM bortezomib. Each dot represents a different sgRNA. The dotted line represents 1%.
   e. sgRNA hits (>1%) identified in the bortezomib surviving cells. Sequences were obtained by next generation sequencing analysis. sgRNAs targeting the target gene of bortezomib, *PSMB5*, are highlighted in bold.
   f. sgRNAs present in single-cell derived clones obtained from the pool of mutagenized and bortezomib resistant cells. Note that the *RXRB* targeting sgRNA always co-occurs with a *PSM85* sgRNA and that every type of clone detected contains an sgRNA targeting *PSMB5.* (*Sequence IDs: 33-37*)
   g. Cell viability of single-cell derived clones containing PSMB5 sgRNAs in the presence of increasing concentrations of bortezomib. Relative cell viability compared to the DMSO treated cells is shown and data points represent means and error bars indicate standard deviation (N=3).
   h. Validation of individual sgRNAs. Two days after transfection. HAP1 cells stably expressing Cas9 were treated with 30 nM bortezomib. Surviving colonies were then counted using an IncuCyte ZOOM.
   i. PSMB5 mutations present in the pool of bortezomib resistant cells.

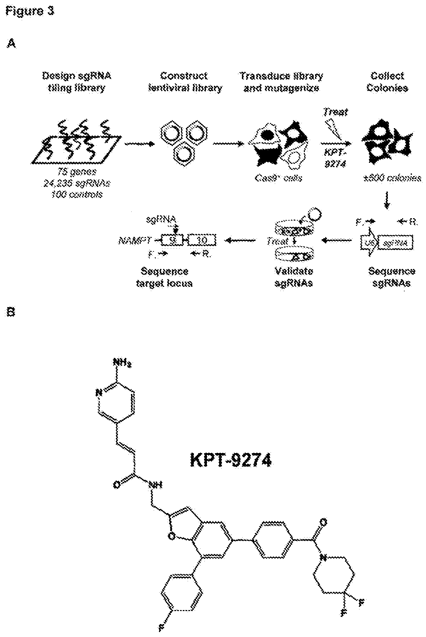

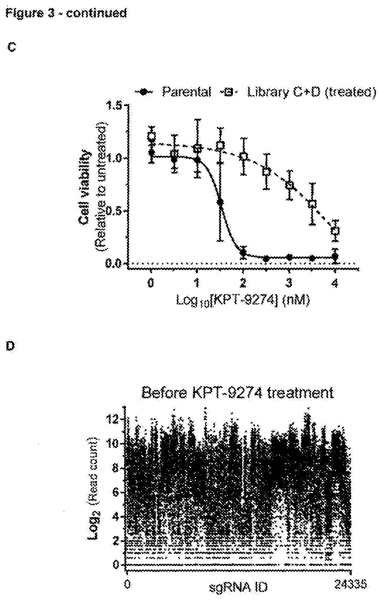

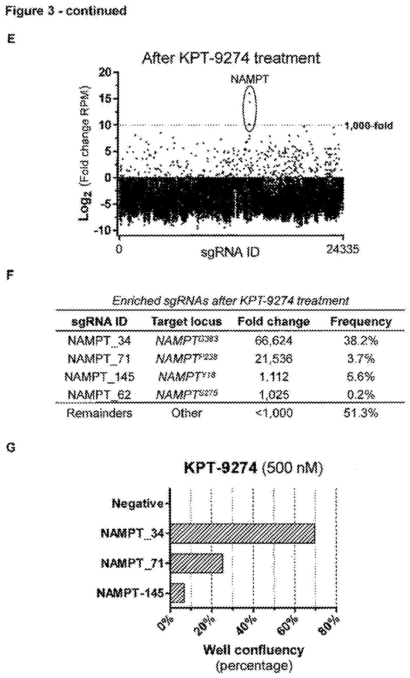

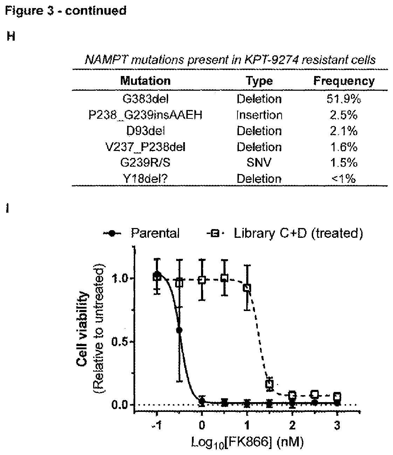

Figure 3 - Application of the CRISPR mutagenesis scanning method for target identification of KPT-9274 using a large-scale CRISPR/Cas9 tiling library
   a. Representation of the experimental workflow for the CRISPR/Cas9-based target identification screen. A lentiviral sgRNA tilling library covering 75 genes targeted by investigational cancer drugs was constructed and transduced in cells stably expressing Cas9, sgRNA expressing cells were enriched by puromycin selection and subsequently treated with KPT-9274 (300 nM) for 14 days. sgRNAs in the resistant colonies were identified by next generation sequencing. These sgRNAs were then individually validated by transfecting them separately in Cas9 expressing cells. Finally, the genomic locus targeted by the validated sgRNAs was sequenced to identify the resistance conferring mutations in the target gene.
   b. Chemical structure of KPT-9274.
   c. Resistance profile of the cell population that survived the mutagenesis screen after selection with KPT-9274. Cell viability was measured in presence of different concentrations of KPT-9274 and was adjusted to the untreated control. Data points represent means and error bars represent standard deviation (N=3).
   d. Representation of the sgRNA library in transduced cells after selection with puromycin and before treatment with KPT-9274. Each dot represents a single guide RNA.
   e. Representation of the sgRNA library In the resistant pool of cells after treatment with KPT-9274. Each dot represents a single guide RNA and the fold change of each sgRNA is plotted, RPM = read per million.
   f. List of sgRNAs present in the cell population that survived the mutagenesis screen after treatment with KPT-9274.
   g. sgRNAs identified in the screen were individually validated by assessing the ability to induce drug resistance. Each sgRNA was separately transfected and cells were treated with KPT-9274 (500 nM) for 8 days before cell confluency was imaged.
   h. NAMPT mutations detected in the cell population that survived the mutagenesis screen. The complete NAMPT CDS was sequenced.
   i. The cell population that survived the mutagenesis screen with KPT-9274 is cross-resistant to the NAMPT inhibitor FK866. Cell viability was measured in presence of different concentrations of FK866 as compared to wild-type. Data points represent means and error bars indicate standard deviation (N=3).

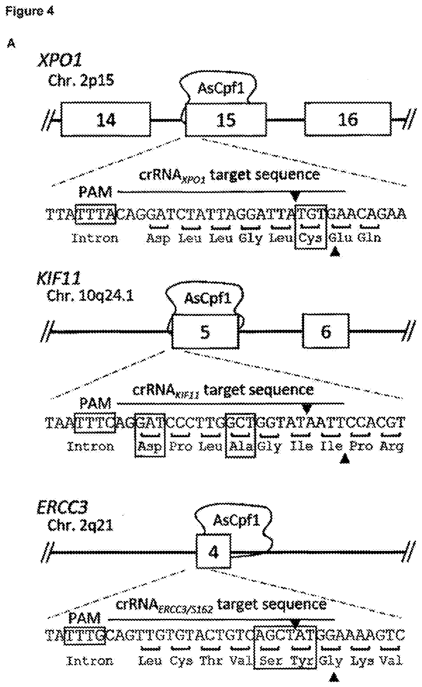

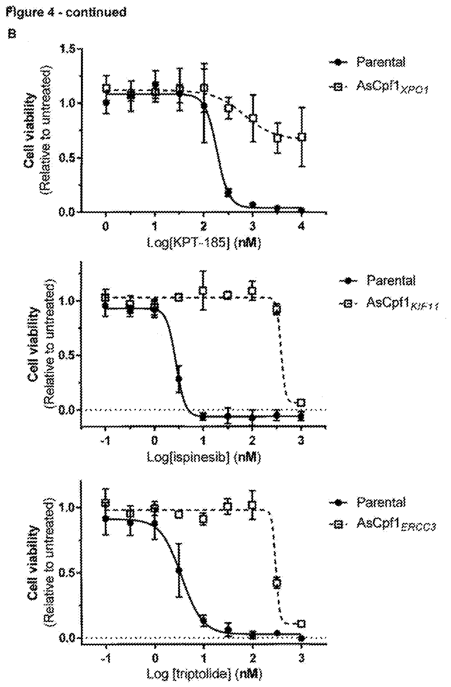

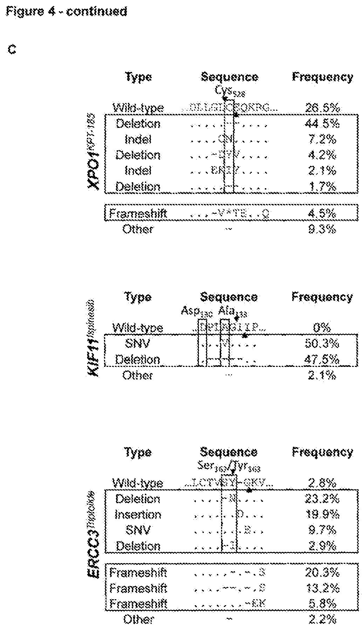

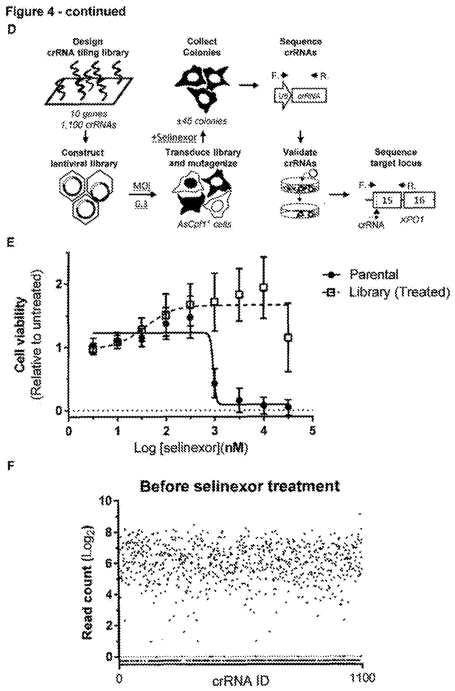

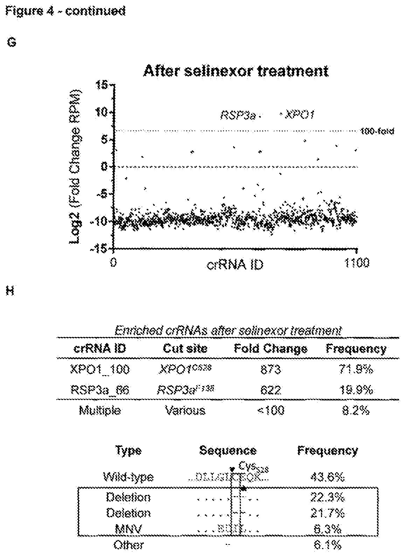

Figure 4 - Rapid selection of resistance mutations generated using the AsCpf1 RNA-guided endonuclease
   a. Overview of AsCpf1 crRNAs targeting *XPO1. KIF11* and *ERCC3* at their resistance hot spot residues. The AsCpf1 cleavage site, generating a four-nucleotide overhang, is denoted by arrowheads. (*Sequence IDs: 38-40,* 50, *63, 64)*
   b. Cell viability of wild-type and mutagenized resistant cells in the presence of increasing concentration of drug. Values are shown relative to the DMSO treated controls. Data points represent means and error bars indicate standard deviation (N=2).
   c. Amino acid sequence variants by targeted amplicon sequencing analysis of the resistant cells by CrispRVariants. Only variants with a frequency ≥1% are shown. *(Sequence IDs: 41-57)*
   d. Overview of the CRISPR/AsCpf1 tiling crRNA library approach used for target identification of selinexor. A lentiviral tilling library targeting 10 different genes was constructed and transduced in AsCpf1 stably expressing cells, which were first selected with puromycin and subsequently treated with selinexor (2 µM). crRNAs present in resistant colonies were identified by next generation sequencing after which the genomic locus targeted by these crRNAs was sequenced to identify the resistance conferring mutations in the target gene.
   e. Cell viability in presence of different concentrations of selinexor of wild-type and resistant cells obtained after library transduction and selinexor treatment. Data points represent means and error bars indicate standard deviation (N=2).
   f. Representation of the different crRNAs present in the cells before treatment with selinexor (after puromycin selection). Each dot represents a different crRNA.
   g. Representation of the crRNAs present in the resistant cell pool after the mutagenesis screen and treatment with selinexor. Each dot represents a different crRNA and the fold change of each crRNA is plotted. RPM = reads per million.
   h. Overview of the enriched crRNAs and the amino acid variants detected in the *XPO1* C528 locus of the selinexor resistant cells. The crRNA cleavage site is shown by arrowheads. (*Sequence IDs: 58-60)*

### EXAMPLES

The identification of the molecular target of small molecule hits identified out of phenotypic screens still remains a major challenge in the drug discovery and development pipeline. While the identification of mutations that confer resistance to a bioactive molecule is recognized as the gold standard proof of its target, selection of drug resistance and subsequent deconvolution of relevant mutations is still a cumbersome task. White many cancer drugs target genetic vulnerabilities, loss-of-function screens fail to identify essential genes in drug mechanism of action because, logically, inactivation of an essential gene causes a lethal phenotype by itself precluding the selection and identification of essential genes as target using these loss-of-function screens. Here we report a new CRISPR-based genetic screening approach using large tiling libraries to rapidly derive and identify drug resistance mutations in essential genes. We validated the approach using ispinesib and bortezomib and applied it as target discovery approach to the novel anticancer agent KPT-9274.

Identifying the cellular target of a chemical hit with valuable activity is a crucial step in drug discovery and development⁵. However, unravelling the molecular target of small molecules remains a challenging, laborious and complex process. Although target deconvolution methods^{6.7} such as chemical proteomics have successfully been applied, they often reveal more than one plausible candidate target protein and carry the risk of identifying interactions that are not related to the compound's activity. The gold standard proof of a small molecules direct target is the discovery of functional mutations that confer resistance in a human cellular context. Therefore, genetic screens are very powerful tools for drug mechanism of action studies⁸. However, current screens either are not well suited to identify essential genes in drug mechanism of action or require whole exome sequencing combined with complex bio-informatics to deconvolute the relevant drug resistance conferring mutations. For example, loss-of-function approaches have been applied to obtain drug resistance⁹⁻¹², but innately lack the ability to detect essential proteins as the direct target for a certain drug, because logically inactivation of essential genes causes a lethal phenotype by themselves precluding selection and identification of essential genes as target using these loss-of-function screens. Because many cancer drugs target essential proteins there is a need for a method that can easily generate and identify drug resistance mutations in essential genes. While classical step-wise drug resistance selection in cancer cells is laborious and often results in off-target multi-drug resistance, genetic screening using chemical induction of single-nucleotide variants has been effectively performed in mammalian cells⁴. However, this chemical mutagenesis approach requires haploid cells and is therefore restricted to drugs active in these cell types. Another bottleneck of general random mutagenesis approaches is the discovery of the resistance mutations. It requires whole-exome sequencing¹⁻⁴ of the human's large genome and the genomic heterogeneity of the cell line makes the deconvolution of the relevant resistance conferring mutations especially challenging. As such, the field would greatly benefit from an approach that can accelerate the drug resistance selection process and simplify subsequent identification of the relevant drug resistance mutations.

Drawing a parallel to the use of UV-mediated double strand breaks (DSBs) to enhance mutagenesis, we reasoned that introduction of DSBs by targeted endonucleases, such as Cas9, and the subsequent error-prone repair via non-homologous end-joining (NHEJ) may be exploited for rational protein mutagenesis to facilitate drug resistance selection. We further hypothesized that large-scale CRISPR sgRNA gene tiling libraries may be applied as a screening approach in cancer cells to identify the molecular target of a chemical inhibitor.

To develop the method, we first designed sgRNAs targeting known resistance hotspots in genes sensitive to three cancer drugs: selinexor, a XPO1 inhibitor, ispinesib, an antineoplastic kinesin-5 (KIF11) inhibitor and triptolide, an anti-proliferative agent targeting ERCC3 (**Fig. 1a** and **b**) The respective sgRNAs were expressed together with Cas9 in the chronic myeloid leukemia derived HAP1 cell line and treated with 4 different concentrations of the corresponding drug. Within a few days of treatment colonies that were resistant to the drugs appeared on the culture plates (**Fig. 1c** and **d**). Next-generation sequencing of the targeted hotspot loci of these resistant colonies revealed known as well many novel resistant protein variants (**Fig**. **1e**). Mutations were mainly localized within 17bp upstream of the Cas9 cleavage site and consisted of deletions and missense mutations (**Fig. 1f**). For XPO1, more than 40 different variants containing a mutation or deletion of the critical C528 residue^{13.14} were detected (**Fig. 1e**), For KIF11 the majority of the reads contained a 9-base pair deletion from codon D130 to L132 (**Fig. 1e**). Interestingly, in contrast to the selinexor resistance mutations, almost all ispinesib resistant sequence alterations were deletions. For ERCC3. we mainly identified in-frame insertions and some deletions between codons K165 and K167 (**Fig**. **1e**). To validate the drug resistance mutations we reinstalled one of the major protein variants (XPO1^{C528S,E529V,Q530H,K531}; KIF11^{L132A}; ERCC3^{V155_K167inst}) into their native locus in parental cells using CRISPR/Cas9-mediated homology-directed repair (HDR), and confirmed that these cells were resistant to the respective drugs.

To demonstrate that this mutagenesis methodology can be broadly applied to other cell types, similar results were obtained in the acute pro-myelocytic leukemia HL-60 and colon cancer HCT 116 cell lines. Taken together, these results demonstrate that spontaneous genetic variation in functionally essential proteins generated during NHEJ repair at the locus of CRISPR/Cas9-mediated DSBs can be exploited to significantly accelerate the selection of drug resistance.

To demonstrate this approach can be applied as a screening method to directly identify the molecular target of a chemical inhibitor, we designed two complementary lentiviral tiling sgRNA libraries that target genes that are modulated by an FDA-approved anti-neoplastic drug (FDA_target library, 115 genes, divided in two subpools A and B of each ±20,000 sgRNAs) or by antineoplastic drugs currently under investigation (investigational_target library; 75 genes, divided in two subpools C and D containing ±12,000 sgRNAs each). These tiling sgRNA libraries contain all possible NGG PAM sites in the exons of the target genes. As a first validation of the methodology, we applied subpool B (containing sgRNAs targeting *PSMB5*) to the proteasome inhibitor bortezomib. HAP1 cells expressing Cas9 were transduced with this library and treated with bortezomib for 14 days (**Fig. 2a**). Surviving colonies were resistant to bortezomib treatment (**Fig. 2b**). Documentation of the sgRNAs present above 1% in the resistant cell pool revealed mainly sgRNAs targeting known *PSMB5* resistance hotspots (**Fig**. **2c****-e**). Validation of the sgRNAs revealed that *PSMB5* was the sole gene that, when mutagenized, conferred bortezomib resistance (**Fig. 2f-h**). Sequencing of the genomic region targeted by the *PSMB5* sgRNAs identified mutations in *PSMB5* (**Fig. 2i**) most of which are similar to known drug resistance mutations and map to the bortezomib binding site. These results effectively validate this tiling library and demonstrate the feasibility of the Cas9-directed mutagenesis scanning strategy for target identification on a larger scale.

Next, to further demonstrate the strength of the method as target identification tool, we applied this approach to the clinical stage anticancer compound KPT-9274, an orally bioavailable small molecule with potent activity against different cancer types. Chemical proteomics has revealed that KPT-9274 interacts with the p21-associated kinase 4 (PAK4) and also inhibition of NAD biosynthesis has been reported. However, the causal association of these activities with cancer cell sensitivity has not been directly demonstrated. We therefore applied library D of our investigational_target tiling library to KPT-9274 (**Fig. 3a**). Colonies that were resistant to KPT-9274 (**Fig. 3b****-c**) appeared within 7-14 days post treatment. Seven sgRNAs, of which 3 targeted nicotinamide phosphoribosyl transferase (*NAMPT*)*,* were enriched in the resistant cell pool (**Fig. 3d****-e**), The *NAMPT* targeting sgRNAs accounted for more than half of the enriched sgRNAs identified (**Fig. 3f**) and these could be confirmed to confer resistance when transfected separately (**Fig. 3g**). Sequencing of the *NAMPT* target loci revealed 7 in-frame mutations (G383del, P238_G239insAAEH, D93del, V237_P238del, G239R, G239S and Y18del) (**Fig. 3h**). We further validated some of these mutations by reinstalling them in their native locus of parental cells by CRISPR-induced HDR. These HDR-edited cells were resistant to KPT-9274 treatment, and were also cross-resistant to the known NAMPT inhibitor FK866, further pinpointing NAMPT as the key cellular target of KPT-9274. These results were confirmed in the pancreas carcinoma MIA Paca2, OPM-2 and myelogenous leukemia K562 cells by transfecting the individual NAMPT sgRNAs conferring KPT-9274 resistance. In addition, the original KPT-9274 resistant cell pool was also cross-resistant to FK866 (**Fig. 3i**) praviding further evidence for NAMPT as the prime target of KPT-9274. Next, to further unambiguously validate our results and corroborate the validity of the conclusions taken from this CRISPR mutagenesis scanning target identification method, we co-crystallized a NAMPT dimer in complex with KPT-9274 (PDB: 5NSD) and explained the identified resistance mutations by modelling some of them into the structure. Altogether, these findings clearly pinpoint NAMPT as the primary target of KPT-9274 and illustrate the power of the described method.

Finally, this CRISPR/Cas9-scanning-target-identification approach may be limited by the availability of NGG PAM motifs at or nearby the resistance hot spot of the investigated drug. To mitigate this restraint, and to orthogonally validate the strategy, we demonstrate the approach is compatible with other endonucleases recognizing a different PAM sequence such as the class 2 type V CRISPR-Cas AsCpf1. We selected AsCpf1 crRNAs targeting TTTN motifs around the same codons for *KIF11, ERCC3* and *XPO1* as described above for Cas9 (**Fig. 4a**). These were transfected along with AsCpf1 in HAP1 cells that were treated with respective compounds. Colonies that formed within a few days of treatment were drug resistant to the specific treatment (**Fig. 4b**) and contained mutations at the known hot spots (**Fig. 4c**). To demonstrate the scalability of the AsCfp1-adapted CRISPR-mutagenesis-target-identification approach to multiple candidate target genes, we next designed a lentiviral tiling crRNA library, similar to the Cas9 tiling library, spanning all possible TTTN PAM sites in the exons from 10 genes (1,100 crRNAs) and applied it to selinexor (**Fig. 4d**). Colonies rapidly appeared and were resistant to selinexor (**Fig. 4e**). One crRNA targeting codon C528 in *XPO1* (**Fig. 4f****-h**) was highly enriched (71.9%) in the resistant cells. Next generation sequencing of the *XPO1* locus revealed protein variants that mainly consisted of a deletion of residues C528 and E529 (**Fig. 4h**), identical to what we observed for the single crRNA (**Fig. 4c**). One other crRNA targeting *RPS3a* was also enriched, but could not be validated when transfected individually. Single-cell derived clones from the original resistant cell pool revealed that this *RPS3a* crRNA always co-appeared with the *XPO1^{C526}* crRNA, suggesting that some cells were transduced with two lentiviral particles; decreasing the MOI will reduce the false positive rate. These results demonstrate that the CRISPR-based mutagenesis scanning approach is compatible with AsCpf1 endonuclease, showing that it can be tailored to other CRISPR endonudeases.

An important strength of this targeted mutagenesis scanning method is that the single guide RNA sequences directly annotate the genomic sequence containing the drug resistance-conferring mutations, avoiding the need for large whole-exome sequencing and complex deconvolution endeavours to uncover the relevant resistance mutations. We also found that resistant cells were commonly hemizygous for the resistance mutation, allowing the approach to uncover recessive mutations and avoiding the need for haploid cells. This is illustrated by triptolide resistance, for which presence of the wild-type allele is sufficient for sensitivity. in multiploid HCT 116 and HL-60 cells.

Recently, the targeting of cytidine deaminases with nuclease-deficient Cas9 has been shown to induce site specific mutagenesis without introducing a DSB to obtain drug resistance ^{15, 16}. These dCas9-cytidine deaminase fusion approaches are complementary to the here described NHEJ-based mutagenesis approach because the mutation spectra clearly differ between both systems. Although the base editing techniques can cover a mutational hotspot region of about 100 bases, they are limited to the introduction of an average of 1.32 base substitutions per read¹⁵. The NHEJ-based approach described here covers a smaller hotspot region but generates larger regions of genetic variation up to 17 bases per read. Furthermore, we observed that in-frame insertions and deletions provide a major mechanism for drug resistance, even when localized to functionally important protein domains, which cannot be obtained by the dCas9-cytidine deaminase fusions in these studies^{15, 16}.

Altogether our findings demonstrate that the localized genetic variation generated by CRISPR-mediated NHEJ repair can be exploited to screen essential genes for gain-of-function mutations. We establish this mutagenesis scanning approach as a genetic screen to identify the molecular target of chemical compounds inhibiting essential proteins and is therefore complementary to the loss-of-function screens. This genetic screen can either be applied on a list of candidate genes identified after a first round of target deconvolution or it can be applied on a predefined shortlist of targets of interest. Indeed, it allows to rapidly select from a primary screen those hit molecules that target a protein or pathway of interest. Nevertheless, even when absolutely no a *priori* knowledge on a potential target of a hit molecule is available, the current format of the method allows coverage of all essential genes with 20-30 tiling libraries for target discovery. Finally. we have illustrated the application of this genetic screen for the identification of drug-target interactions using cellular toxicity as phenotypic selection, but it may also be applicable to other phenotypic reporter assays.

### Methods

### Cell culture

HAP1 cells were obtained from Horizon Discovery. HCT 116, MIA PaCa2 and K-562 cells were obtained from ATCC, HL-60 cells from Sigma Aldrich, and OPM-2 from DSMZ. spCas9 and asCpf1 expressing cells were generated in house. HAP1, HL-60 and K-562 cells were grown and passaged every 2-3 days in IMDM. HCT 116 cells were grown in McCoy's 5A medium, MIA PaCa2 cells were cultured in DMEM and OPM-2 in RPMI 1640. All media were supplemented with 10% fetal bovine serum and 20 µg/m gentamicin. Cells were incubated at 37°C and 5% CO₂.

### Compounds

KPT-185, selinexor (KPT-330), and KPT-9274 were provided by Karyopharm Therapeutics (Newton, MA), Ispinesib, triptolide, bortezomib and FK866 were obtained from SelleckChem. All compounds were dissolved in DMSO, except for FK866, which was dissolved in ethanol.

### DNA constructs

The plasmid expressing humanized spCas9 was obtained from Labomics. The plasmid expressing humanized asCpf1 was obtained from Addgene (69982). Plasmids containing sgRNAs or crRNAs were cloned in house. The pLCKO vector used for generation of the lentiviral library was obtained from Addgene (73311). Single-stranded DNA oligonucleotides for use with HDR were obtained from Integrated DNA Technologies.

### Generation of stable Cas9 and AsCpf1 HAP1 cell lines

Knock-in HAP1 cell lines stably expressing apCas9 or AsCpf1 were generated using the CRISPaint principle¹⁷. Briefly, cells were electroporated with the Neon Electroporation System (Thermo Fisher Scientific) using a 10 µL, 1,450V and 3 pulses of 10 ms in Buffer R (Neon Electroporation Kit) with a plasmid encoding a sgRNA targeting the C-tarminus of *SDHA (250 ng),* a plasmid encoding Cas9 and a sgRNA targeting the donor plasmid (250 ng) and a repair donor plasmid containing a PAM and sgRNA targeting site and the sequence for P2A-mCherry-T2A-Cas9-P2A-HygroR or T2A-AsCpf1-P2A-HyrgoR (250 ng) to stably integrate spCas9 or asCpf1 downstream of the *SDHA* housekeeping gene. Cells were plated in a 6-well plate and two days after transaction, cells were selected with 300 µg/mL hygromycin B for a period of 10 days and then checked for expression of the red fluorescent mCherry. spCas9 or AsCpf1 endonuclease activity was assessed in the polyclonal mixture by indel detection in *XPO1* after sgRNA/crRNA transfsction in the respective cell line.

### Transfection of single guide RNAs, crRNAs and HDR templates

Cells were transfected with the Neon Electroporation System after resuspension in Buffer R. DNA plasmids expressing the spCas9 or asCpf1 endonuclease and guiding RNA were added to a concentration of 37.5 ng/µL per plasmid and electroporated at 1,400-1,475V with 3 pulses of 10 ms. Two to three days after transaction, cells were treated with the respective drug to select for resistance over 7-14 days. Colonies were imaged with an incuCyte© ZOOM (Essen Bioscience).

For HDR, a 123-134 bases long ssDNA oligonucleotide (850 ng) was added to the electroporation mixture in addition to the respective sgRNA and spCas9 expressing plasmids. Two days after electroporation, cells were treated with respective drugs for 5 days before imaging with an IncuCyte© ZOOM (Essen Bioscience). Following imaging, the HDR-template transfected cells were grown under drug selection for an additional week before any further experiments were performed.

### Cell viability assays

Cell viability allays were performed by plating 3,000 HAP1, 5.000 HCT 116 or HL-60 cells in 96-well plates containing DMSO or a dilution of the test compound. Cells were incubated for 72 hours at 37°C and 5% CO₂. Cell viability was then assessed with the CellTiter 96® AQueous Non-Radioactive Cell Proliferation Assay (Promega) and colorimetric signals were measured with a Safire2™ (TECAN). Assays were performed in triplicate and each experiment was repeated at least once. Obtained values were adjusted with the background signal and divided by the DMSO control. Relative data values were then visualized and analyzed using a log-based 4 parameter model (GraphPad Prism).

For single guide drug resistance validation assays, 125.000 cells were transfected with Cas9 and the individual sgRNAs using the Neon Electroporation system as described above and plated into 6-well plates. Cells were treated 2-3 days after transfection with the respective compound for a period of 5-7 days, the medium was regularly refreshed and dead cells were washed away before imaging confluency using a live cell analysis system (Essen Bioscience, IncuCyte ZOOM®).

### DNA extraction and sequencing

Genomic DNA was isolated from 1 million cells with the QIAamp DNA mini kit using RNase A. For Sanger sequencing, the region of interest was amplified by PCR with the CloneAmp HiFi PCR premix (Ctontech). The amplified DNA was then purified (QIAquick PCR purification kit (Qiagen)) and sequenced (Macrogen). For targeted amplicon sequencing, the region of interest (*KIF11_{A133}, XPO1_{C528}, ERCC3_{D54}, ERCC3_{S182}, NAMPT_{Y1B}. NAMPT_{S240} or NAMPT_{G383}*) was first amplified over 24 cycles in 25 µL PCR reactions containing 50 ng genomic DNA with the Phusion® High-Fidelity PCR Master Mix with HF Buffer (NEB) and with custom primers containing adapter regions for Nextera indexes (IDT). Amplified DNA was purified and 1.5-2 µL of this DNA was PCR amplified over 25 cycles with CloneAmp HiFi PCR Premix (Clontech) using indexing primers containing P5 and P7 Illumine adapters in 25 µL reactions to index the samples. Indexed samples were purified using magnetic Agencourt AMPure XP beads (Beckman Coulter) and eluted in TE buffer. Samples were then diluted to 2-4 nM and pooled to form the initial library. This library was then denatured and diluted according to the instructions for paired-end sequencing on a MiSeq (Illumina) with a MiSeq V2-300 or 500 cycles kit (Illumina) and 10% PhiX v3 (Illumina) spike-in. For a list of primers see the supplemental data file.

### Analysis of next-generation sequencing data

FastQ files obtained after MiSeq sequencing were demultiplexed with the MiSeq Reporter software (Illumina). Demultiplexed and paired reads were trimmed, filtered and then aligned to the reference amplicon in Geneious (v9. Biomatters). To obtain haplotypes present in drug-resistant samples, bam files were analyzed with the CrispRVariants package run in RStudio by defining a 35-50 bp spanning region across the endonuclease cut site, as defined by pre-analysis of localized variants within Geneious. For this purpose, the CrispRVariants "readtotarget" input was run with parameters "upstream.snv" (30) and "downstream.snv" (15) on corresponding paired end sequencing reads to allow for haplotype determination. Haplotype nucleotide sequences were extracted with a small script. Nucleotide sequences were then visualized and mapped to the reference in Geneious v9 (Biomatters) and amino acid variants were determined. For visualization of the spectra of single nucleotide variants, NGS reads were aligned to the reference gene. Nucleotide occurrence frequencies were then determined in R on the aligned NGS reads using the deepSNV Bioconductor package. Sequences containing sgRNA/crRNAs from the lentiviral screens were trimmed from adapter sequences. Individual sgRNA/crRNA sequencing reads were then aligned to the pLCKO-U6-sgRNA vector within Geneious, counted with MaGeCK¹⁸ and visualized in GraphPad Prism.

### Cloning of the sgRNA and crRNA libraries

The 2209 sgRNA sequences used in the ispinesib-KIF11 pilot screen were obtained by selecting all N₂₁GG sequences available in the NCBI consensus coding sequences of the main isoforms of 9 genes (*KIF11, XPO1, ERCC3, PAK4, ABL1, TUBB, ACTB, RSP3a* and *H2BFM2*). Also included were 100 control sgRNAs and all sgRNA sequences were appended 5' and 3' with small DNA sequences to facilitate PCR (total length 60 nt).

To obtain the AsCpf1 crRNA sequences, the coding sequence of the main isoforms of the 10 genes (*KIF11, XPO1, ERCC3, PAK4, ABL1, TUBB, ACTB, RSP3a, H2BFM2* and *p53*) were extracted from NCBI. For each intron-exon boundary 25 nucleotides were added to the exonic sequences. From these sequences, all TTTN₂₄ sequences were extracted and appended 5' with the AsCpf1 direct repeat backbone (TAATTTCTACTCTTGTAGA, SEQ ID 65) and thirty scrambled control crRNAs were included. Sequences were then further appended 5' and 3' with small DNA sequences to facilitate PCR (total length 79 nt).

The sgRNA sequences for the "FDA-target" and "non-FDA-target" libraries were obtained with a custom script run in RStudio. In brief, target genes for the libraries were roughly determined by the drug target list available for approved and investigational antineoplastic agents on the Kyoto Encyclopedia of Genes and Genomes (KEGG) database (retrieved July 2016) combined with a small literature study. The target list was then filtered from agents consisting of analogues of nucleotide or metabolic products. The web-based Biomart (Ensemble) was used to obtain the start and end coordinates of all CDS exons retrieved from the NCBI refseq entries available for all isoforms of the predefined target genes. Twenty base pairs were added 5' and 9 base pairs were added 3' to each of the exonic start or end coordinates on the forward and reverse strands respectively to include sgRNAs located on exon-intron boundaries. These expanded and strand specific coordinates were used to search through the NCBI reference sequences to obtain all N₂₁GG sequences within these coordinates on both the forward and reverse strand. Duplicate sgRNA sequences were removed on a gene-per-gene basis and sgRNAs were then appended with additional sequences to facilitate PCR and the generation of subpools. See the supplementary data file for the gene target lists and the individual sgRNA sequences.

All appended sgRNA and crRNA sequences were synthesized as pools by Customarray Inc. (Bothell, WA) on a 12K (9/10 gene libraries) or 90K ("non-FDA-target"/"FDA-target") chip. The sgRNA/crRNA pools were amplified in 10 parallel reactions (25 µL, 1 ng input) by PCR with the CloneAmp HiFi premix kit (Clontech) and PCR products were purified with the QIAQuick Nucleotide Removal kit (QiaGen). The purified PCR products were then subjected to restriction digestion (6 parallel reactions) with BfuAl (NEB) overnight at 50°C. After digestion, 6 ligation reactions containing 33 ng of digested sgRNA/crRNAs and 500 ng of the BfuAl and Nsil predigested pLCKO vector were performed overnight at 16°C with T4 DNA ligase (NEB). The pooled mixture of ligated pLCKO vectors was then purified with the QIAquick nucleotide removal kit (Qlagen) and electroporated into Endura competent cells (Lucigen) with a Gene Pulser system (Biorad) according to the manufacturer's instructions. Transformed cells were then plated in 15cm-diameter petridishes containing prewarmed LB agar with 100 µg/mL ampicilin and grown overnight at 32°C. The following day colonies were counted and a fold representation of 400 ("FDA"" libraries A and B). 2,700 ("non-FDA" libraries C and D), 30,000 (9 gene Cas9 library) or 90,000 (AsCpf1 library) was estimated. All colonies were pooled per library for plasmid extraction with the PureLink® HiPure Plasmid Maxiprep (Invitrogen).

### Lentiviral library

The pooled and purified pLCKO-U6-sgRNA/crRNA plasmid libraries were provided to Applied Biological Materials Inc. (Richmond, BC, Canada) to generate lentiviral particles coated with the VSV-G protein and containing the desired genetic information for human expression of the sgRNA/crRNAs. Viral stocks were titrated on wild-type HAP1 cells to determine the multiplicity of infection (MOI).

### Drug-target identification screens

HAP1 cells stably expressing spCas9 or asCpf1 were resuspended in supplemented IMDM containing 8 µg/mL polybrene and transduced with lentiviral particles containing the desired sgRNA/crRNAs at a MOI of 0.25 (coverage of 5,000x per sgRNA for spCas9) or 0.35 (coverage of 15,000x per crRNA for asCpf1) by spinfection in 12-well plates. The next day cells were transferred to T150 cell flasks and selected with 1 µg/mL puromycin. Then 4 million (ispinesib/selinexor) or 10 million (KPT-9274/bortezomib) cells were harvested for DNA extraction and the remaining cells were treated for a period of 2 weeks with 8 nM ispinesib, 30 nM bortezomib, 300 nM KPT-9274 or 2 µM selinexor. The compound-containing medium was regularly refreshed. After treatment, surviving cells were harvested and the DNA was extracted which was subjected to 5-10 parallel 100 µL PCR reactions (24 cycles, 2,000 ng per reaction) with pLCKO primers carrying Nextera adapter sequences and using the Phusion High-Fidelity PCR mastermix with HF buffer (NEB). Amplified DNA was purified and pooled and a second PCR was performed over 24 cycles on 50 ng with Nextera indexing primers (Illumina). Further processing for next generation sequencing analysis was performed as described above.

### SEQUENCE LIST

SEQ ID 1: dna; GGATTATGTGAACAGAAAAGAGGC
SEQ ID 2: dna: taatttcagGATCCCTTGGCTGGT
SEQ ID 3: dna: CAGCTATGGAAAAGTCAAGCTGGTC
SEQ ID 4: prt: GLCEQKRGKDN
SEQ ID 5: prt: GLSVHIRGKDN
SEQ ID 6: prt: GYVNRKRGKDN
SEQ ID 7: prt: GYRGKDN
SEQ ID 8: prt: GDPPVRGKDN
SEQ ID 9: prt: GLCEQKKRQRX
SEQ ID 10: prt: GLCEQKEAKII
SEQ ID 11: prt: GLCEQRQRX
SEQ ID 12: prt: OPLAGIIP
SEQ ID 13: prt: AGIIP
SEQ ID 14: prt: DPAGIIP
SEQ ID 15: prt: DPFLAGIIP
SEQ ID 16: prt: DGIIP
SEQ ID 17: prt: DPGIIP
SEQ ID 18: prt: DLAGIIP
SEQ ID 19: prt: DPAGIIP
SEQ ID 20: prt: DPFGWYNS
SEQ ID 21 prt: DPWYNS
SEQ ID 22: prt: DPLLXFQAGIIP
SEQ ID 23: prt: SYGKVKLV
SEQ ID 24: prt: SYGKVLKLV
SEQ ID 25: prt: SYGKVXQLV
SEQ ID 26: prt: SYGKLV
SEQ ID 27: prt: SYGKVXKLV
SEQ ID 28: prt: SYGKAMEKLV
SEQ ID 29: prt: SYGKV
SEQ ID 30: prt: SYGKVVKLV
SEQ ID 31: prt: SYGNVKLV
SEQ ID 32: prt: SYGKVTXQLV
SEQ ID 33: dna: GTAAGCACCCGCTGTAGCCC
SEQ ID 34: dna: TGGGCTCGGTGCTGCCCTAC
SEQ ID 35: dna: CACCATGGCTGGGGGCGCAG
SEQ ID 36: dna: CGGCATGCCGCAGGGCAGTG
SEQ ID 37: dna: CAGTACTCAGCCTGGCAAGG
SEQ ID 38: dna: ttatttacagGATCTATTAGGATTATGTGAACAGAA
SEQ ID 39: dna: taatttcagGATCCCTTGGCTGGTATAATTCCACGT
SEQ ID 40: dna: tatttgcagTTGTGTACTGTCAGCTATGGAAAAGTC
SEQ ID 41: prt: DLLGLCEQKRG
SEQ ID 42: prt: DLLGLQKRG
SEQ ID 43: prt: DLLGCNEQKRG
SEQ ID 44: prt: DLLDYVQKRG
SEQ ID 45: prt: DLLEKIYQKRG
SEQ ID 46: prt: DLLGLQKRG
SEQ ID 47: prt: DLLVXTEKRQ
SEQ ID 48: prt: DPLAGIIP
SEQ ID 49: prt; DPLVGIIP
SEQ ID 50: prt: LCTVSYGKV
SEQ ID 51: prt: LCTVNGKV
SEQ ID 52: prt: LCTVSYDGKV
SEQ ID 53: prt LCTVSYEKV
SEQ ID 54: prt: LCTVIGKV
SEQ ID 55: prt: LCTVSKS
SEQ ID 56: prt: LCTVKS
SEQ ID 57: prt: LCTVSYEK
SEQ ID 58: prt: DLLGLCEQK
SEQ ID 59: prt: DLLGLQK
SEQ ID 60: prt: DLLEDILQK
SEQ ID 61: prt: GLCEQKRG
SEQ ID 62. prt: DPLAG
SEQ ID 63: prt: DLLGLCEQ
SEQ ID 64: prt: DPLAGIIPR
SEQ ID 65: dna: TAATTTCTACTCTTGTAGA

### References

1. Wacker, S.A., Houghtaling, B.R., Elemento, O. & Kapoor, T.M. Using transcriptome sequencing to identify mechanisms of drug action and resistance. Nat Chem Biol 8, 235-237 (2012).
2. Kasap, C., Elemento, O. & Kapoor, T.M. DrugTargetSeqR: a genomics- and CRISPR-Cas9-based method to analyze drug targets. Nat Chem Biol 10. 626-628 (2014).
3. Smumyy, Y. et al. DNA sequencing and CRISFR-Cas9 gene editing for target validation in mammalian cells. Nat Chem Biol 10, 623-625 (2014).
4. Forment, J.V. et al. Genome-wide genetic screening with chemically mutagenized haploid embryonic stem cells. Nat Chem Biol 13, 12-14 (2017).
5. Bunnage, M.E., Gilbert, A.M., Jones, L.H. & Hett, E.C. Know your target, know your molecule. Nat Chem Biol 11, 368-372 (2015).
6. Schenone, M., Dancik, V., Wagner, B.K. & Clemons, P.A. Target identification and mechanism of action in chemical biology and drug discovery. Nat Chem Biol 9, 232-240 (2013).
7. Terstappen, G.C., Schlupen, C., Raggiaschi, R. & Gaviraghi, G. Target deconvolution strategies in drug discovery. Nat Rev Drug Discov 6, 891-903 (2007).
8. Nijman, S.M. Functional genomics to uncover drug mechanism of action. Nat Chem Biol 11, 942-948 (2015).
9. Birsoy, K. et al. MCT1-mediated transport of a toxic molecule is an effective strategy for targeting glycolytic tumors. Nat Genet 45, 104-108 (2013).
10. Burckstummer, T. et al. A reversible gene trap collection empowers haploid genetics in human cells. Nat Methods 10, 965-971 (2013).
11. Shalem, O. et al. Genome-scale CRISPR-Cas9 knockout screening in human cells. Science 343, 84-87 (2014).
12. Wang, T., Wei, J.J., Sabatini, D.M. & Lander, E.S. Genetic screens in human cells using the CRISPR-Cas9 system. Science 343, 80-84 (2014).
13. Neggers, J.E. et al. Identifying drug-target selectivity of small-molecule CRM1/XPO1 inhibitors by CRISPR/Cas9 genome editing. Chem Biol 22, 107-116 (2015).
14. Lapalombella, R. et al. Selective inhibitors of nuclear export show that CRM1/XPO1 is a target in chronic lymphocytic leukemia. Blood 120, 4621-4634 (2012).
15. Hess, G.T. et al. Directed evolution using dCas9-targeted somatic hypermutation in mammalian cells. Nat Methods 13,1036-1042 (2016).
16. Ma, Y. et al. Targeted AID-mediated mutagenesis (TAM) enables efficient genomic diversification in mammalian cells. Nat Methods 13, 1029-1035 (2016).
17. Schmid-Burgk, J.L, Honing, K., Ebert, T.S. & Homung, V. CRISPaint allows modular base-specific gene tagging using a ligase-4-dependent mechanism, Nat Commun 7, 12338 (2016).
18. Li, W. et al. MAGeCK enables robust identification of essential genes from genome-scale CRISPR/Cas9 knockout screens. Genome Bid 15, 554 (2014).

## Claims

1. A method for generating a stimulus resistant cell line, comprising
(i) transduce a cell line, stably expressing an RNA-guided endonuclease of the clustered regularly interspaced short palindromic repeats (CRISPR) system wherein said endonuclease generates mutations by non-homologous end-joining (NHEJ), with a vector library comprising guide RNAs targeting exonic sequences, and targeting intronic sequences within 30 base pairs of an intron-exon boundary, of at least one candidate target gene or the whole exome of the organism the stimulus is targeting,
wherein the method does not comprise the use of a homology-directed repair (HDR) template or substrate;
(ii) select for transduced cells at the end of step (i);
(iii) treat selected cells at the end of step (ii) with the stimulus;
(iv) grow the stimulus resistant colonies that are formed at the end of step (iii);
(v) identify or sequence the guide RNA sequence(s) present in the resistant colonies generated in (iv);
(vi) sequence the genomic region around the target sequence of the identified guide RNA(s) to identify the genetic mutations that confer cellular resistance to the stimulus; and
(vii) select those colonies wherein the mutations consist of in-frame insertions and/or in-frame deletions and/or in-frame indels and/or point mutations resulting in functional protein variants, and excluding mutations that introduce a premature stop codon that leads to loss-of-function, of the identified target sequence of step (vi).

2. The method according to claim 1, wherein the RNA-guided endonuclease is selected from the group consisting of CRISPR/Cas9 Cpf1, Cas9-VQR, Cas9-EQR, Cas9-VRER, AsCpf1-S542R/K607R, AsCpf1-S542R/K548V/N552R, LbCpf1-G532R/K595R, LbCPf1-G532R/K538V/Y542R.

3. The method according to claim 1 or 2, wherein the vector library is a tiling library.

4. The method according to any of claims 1 to 3, wherein the selection step (ii) and/or step (iii) is based on the selection of surviving clones or on another selectable or enrichable phenotype.

5. The method according to any of claims 1 to 4, wherein the stimulus is a bioactive molecule with anticancer activity or a bioactive molecule inducing a selectable or enrichable phenotype.

6. The method according to any of claims 1 to 5, wherein the stimulus is a drug, a pathogen, a virus or a bacterium.

7. The method according to any of claims 1 to 6, wherein the vector library is a lentiviral vector library, or wherein the vector library comprises all possible guide RNAs present in the coding sequence of at least one candidate target gene or present in the whole exome of the organism the stimulus is targeting.

8. The method according to any of claims 1 to 7, wherein the RNA-guided endonuclease is fused with a DNA-repair enzyme, or wherein the RNA-guide recruits a DNA repair enzyme such as a β-polymerase, θ-polymerase or DNA Ligase IV, including any mutant thereof.

9. The method according to any of claims 1 to 8, wherein the vector library of step (i) comprises a selection marker and wherein in step (ii) transduced cells are selected by using that marker.

10. The method according to claim 9, wherein the marker is an antibiotic resistance marker, and the transduced cells in step (ii) are selected by growing the cells in the presence of said antibiotic.

11. The method according to any of claims 1 to 10, wherein the stimulus is lethal for the untreated, wild type cell and wherein the stimulus is lethal for all the cells in step (iii) which do not comprise a mutation conferring resistance to said stimulus at the end of step (ii).

12. The method according to any of claims 1 to 11, wherein step (i) is about 1 day and/or step (ii) is about 5 days and/or step (iii) is about 1 to 2 weeks and/or step (iv) is about 1 to 2 weeks.

13. The method according to any of claims 1 to 12, wherein the stimulus acts on an essential gene and the target sequence in step (vi) is part of said essential gene.

14. A method for generating a mutant cell line, comprising
(i) transduce a cell line, stably expressing an RNA-guided endonuclease of the clustered regularly interspaced short palindromic repeats (CRISPR) system wherein said endonuclease generates mutations by non-homologous end-joining (NHEJ), with a vector library comprising guide RNAs targeting exonic sequences and targeting intronic sequences within 30 base pairs of an intron-exon boundary, in at least one candidate target gene or in the whole exome of the organism the stimulus is targeting, wherein the method does not comprise the use of a homology-directed repair (HDR) template or substrate ;
(ii) select for the transduced cells at the end of step (i);
(iii) select the cells at the end of step (ii) with a certain phenotype;
(iv) grow the selected colonies at the end of step (iii);
(v) identify or sequence the guide RNA sequence(s) present in the selected colonies generated in (iv);
(vi) sequence the genomic region around the target sequence of the identified guide RNA(s) to identify the mutations that cause said certain phenotype; and
(vii) select those colonies wherein the mutations are in frame insertions and/or in frame deletions and/or point mutations of the identified target sequence of step (vi).

15. The method according to claim 14, wherein the RNA-guided endonuclease is selected from the group consisting of CRISPR/Cas9 Cpf1, Cas9-VQR, Cas9-EQR, Cas9-VRER, AsCpf1-S542R/K607R, AsCpf1-S542R/K548V/N552R, LbCpf1-G532R/K595R, and LbCPf1-G532R/K538V/Y542R.

## Patentansprüche

1. Ein Verfahren zur Erzeugung einer stimulusresistenten Zelllinie, umfassend:
(i) Transduzieren einer Zelllinie, die stabil eine RNA-geführte Endonuklease des Clustered Regularly Interspaced Short Palindromic Repeats (CRISPR)-Systems exprimiert, wobei die genannte Endonuklease Mutationen erzeugt durch nicht-homologe Endverknüpfung (NHEJ), mit einer Vektorbibliothek bestehend aus Guide-RNAs, die gezielt binden an exonische Sequenzen und gezielt binden an intronische Sequenzen innerhalb von 30 Basenpaaren einer Intron-Exon-Grenze mindestens eines Kandidaten-Zielgens oder des gesamten Exoms des Organismus, gegen den der Stimulus gerichtet ist, wobei das Verfahren nicht die Verwendung eines Homology-Directed Repair (HDR)-Templates oder Substrats umfasst;
(ii) Selektieren auf transduzierte Zellen am Ende von Schritt (i);
(iii) Behandeln von selektierten Zellen am Ende von Schritt (ii) mit dem Stimulus;
(iv) Kultivieren der stimulusresistenten Kolonien, die am Ende von Schritt (iii) gebildet werden;
(v) Identifizieren oder Sequenzieren der Guide-RNA-Sequenz(en), die in den in Schritt (iv) erzeugten resistenten Kolonien vorhanden sind;
(vi) Sequenzieren der genomischen Region rund um die Zielsequenz der identifizierten Guide-RNA(s), um die genetischen Mutationen zu identifizieren, die eine zelluläre Resistenz gegen den Stimulus verleihen; und
(vii) Selektieren derjenigen Kolonien, in denen die Mutationen aus In-Frame-Insertionen und/oder In-Frame-Deletionen und/oder In-Frame-Indels und/oder Punktmutationen bestehen, die zu funktionsfähigen Proteinvarianten führen, und Ausschließen von Mutationen, die ein vorzeitiges Stop-Codon einfügen, das zum Funktionsverlust der identifizierten Zielsequenz von Schritt (vi) führt.

2. Das Verfahren nach Anspruch 1, wobei die RNA-geführte Endonuklease ausgewählt wird aus der Gruppe bestehend aus CRISPR/Cas9 Cpf1, Cas9-VQR, Cas9-EQR, Cas9-VRER, AsCpf1-S542R/K607R, AsCpf1-S542R/K548V/N552R, LbCpf1-G532R/K595R, LbCPf1-G532R/K538V/Y542R.

3. Das Verfahren nach Anspruch 1 oder 2, wobei die Vektorbibliothek eine "Tiling Library" ist.

4. Das Verfahren nach einem der Ansprüche 1 bis 3, wobei der Selektionsschritt (ii) und/oder Schritt (iii) auf der Selektion von überlebenden Klonen oder auf einem anderen selektierbaren oder anreicherbaren Phänotyp basiert.

5. Das Verfahren nach einem der Ansprüche 1 bis 4, wobei der Stimulus ein bioaktives Molekül mit Antikrebsaktivität oder ein bioaktives Molekül ist, das einen selektierbaren oder anreicherbaren Phänotyp induziert.

6. Das Verfahren nach einem der Ansprüche 1 bis 5, wobei der Stimulus ein Arzneimittel, ein Pathogen, ein Virus oder ein Bakterium ist.

7. Das Verfahren nach einem der Ansprüche 1 bis 6, wobei die Vektorbibliothek eine lentivirale Vektorbibliothek ist oder wobei die Vektorbibliothek alle möglichen Guide-RNAs umfasst, die in der codierenden Sequenz mindestens eines Kandidaten-Zielgens vorhanden sind oder im gesamten Exom des Organismus, gegen den der Stimulus gerichtet ist, vorhanden sind.

8. Das Verfahren nach einem der Ansprüche 1 bis 7, wobei die RNA-geführte Endonuklease mit einem DNA-Reparaturenzym fusioniert ist oder wobei die Guide-RNA ein DNA-Reparaturenzym wie z. B. eine β-Polymerase, θ-Polymerase oder DNA-Ligase IV, einschließlich jeder ihrer Mutanten, rekrutiert.

9. Das Verfahren nach einem der Ansprüche 1 bis 8, wobei die Vektorbibliothek von Schritt (i) einen Selektionsmarker umfasst und wobei die in Schritt (ii) transduzierten Zellen unter Verwendung dieses Markers selektiert werden.

10. Das Verfahren nach Anspruch 9, wobei der Marker ein Antibiotikumresistenzmarker ist, und die in Schritt (ii) transduzierten Zellen selektiert werden, indem die Zellen in Gegenwart des genannten Antibiotikums kultiviert werden.

11. Das Verfahren nach einem der Ansprüche 1 bis 10, wobei der Stimulus für die unbehandelte Wildtypzelle letal ist und wobei der Stimulus letal ist für alle Zellen in Schritt (iii), die keine Mutation enthalten, welche eine Resistenz gegen den genannten Stimulus am Ende von Schritt (ii) verleiht.

12. Das Verfahren nach einem der Ansprüche 1 bis 11, wobei Schritt (i) etwa 1 Tag und/oder Schritt (ii) etwa 5 Tage und/oder Schritt (iii) etwa 1 bis 2 Wochen und/oder Schritt (iv) etwa 1 bis 2 Wochen dauert.

13. Das Verfahren nach einem der Ansprüche 1 bis 12, wobei der Stimulus auf ein essentielles Gen einwirkt und die Zielsequenz in Schritt (vi) ein Teil des genannten essentiellen Gens ist.

14. Ein Verfahren zur Erzeugung einer mutanten Zelllinie, umfassend:
(i) Transduzieren einer Zelllinie, die stabil eine RNA-geführte Endonuklease des Clustered Regularly Interspaced Short Palindromic Repeats (CRISPR)-Systems exprimiert, wobei die genannte Endonuklease Mutationen erzeugt durch nicht-homologe Endverknüpfung (NHEJ), mit einer Vektorbibliothek bestehend aus Guide-RNAs, die gezielt binden an exonische Sequenzen und gezielt binden an intronische Sequenzen innerhalb von 30 Basenpaaren einer Intron-Exon-Grenze in mindestens einem Kandidaten-Zielgen oder im gesamten Exom des Organismus, gegen den der Stimulus gerichtet ist, wobei das Verfahren nicht die Verwendung eines Homology-Directed Repair (HDR)-Templates oder Substrats umfasst;
(ii) Selektieren auf die transduzierten Zellen am Ende von Schritt (i);
(iii) Selektieren der Zellen am Ende von Schritt (ii) mit einem bestimmten Phänotyp;
(iv) Kultivieren der selektierten Kolonien am Ende von Schritt (iii);
(v) Identifizieren oder Sequenzieren der Guide-RNA-Sequenz(en), die in den in Schritt (iv) erzeugten Kolonien vorhanden sind;
(vi) Sequenzieren der genomischen Region rund um die Zielsequenz der identifizierten Guide-RNA(s), um die Mutationen zu identifizieren, die den genannten bestimmten Phänotyp verursachen; und
(vii) Selektieren derjenigen Kolonien, in denen die Mutationen In-Frame-Insertionen und/oder In-Frame-Deletionen und/oder Punktmutationen der identifizierten Zielsequenz von Schritt (vi) sind.

15. Das Verfahren nach Anspruch 14, wobei die RNA-geführte Endonuklease ausgewählt wird aus der Gruppe bestehend aus CRISPR/Cas9 Cpf1, Cas9-VQR, Cas9-EQR, Cas9-VRER, AsCpf1-S542R/K607R, AsCpf1-S542R/K548V/N552R, LbCpf1-G532R/K595R und LbCPf1-G532R/K538V/Y542R.

## Revendications

1. Procédé de génération d'une lignée cellulaire résistant à un stimulus, comprenant:
(i) transduire une lignée cellulaire, exprimant de manière stable une endonucléase guidée par ARN du système de répétitions palindromiques courtes espacées régulièrement et groupées (CRISPR), ladite endonucléase générant des mutations par jonction d'extrémités non homologues (NHEJ), avec une banque de vecteurs comprenant des ARN guides ciblant des séquences exoniques, et ciblant des séquences introniques à l'intérieur de 30 paires de bases d'une limite intron-exon, d'au moins un gène cible candidat ou de l'exome entier de l'organisme visé par le stimulus, étant entendu que le procédé ne comprend pas l'utilisation d'un modèle ou d'un substrat de réparation dirigée par homologie (HDR) ;
(ii) sélectionner les cellules transduites à la fin de l'étape (i) ;
(iii) traiter les cellules sélectionnées à la fin de l'étape (ii) avec le stimulus ;
(iv) développer les colonies résistantes aux stimuli qui se forment à la fin de l'étape (iii) ;
(v) identifier ou séquencer la ou les séquence(s) d'ARN guides présentes dans les colonies résistantes générées en (iv) ;
(vi) séquencer la région génomique autour de la séquence cible du ou des ARN guide(s) identifié(s) pour identifier les mutations génétiques conférant une résistance cellulaire au stimulus ; et
(vii) sélectionner les colonies dans lesquelles les mutations consistent en des insertions avec maintien du cadre et/ou des délétions avec maintien du cadre et/ou des indels avec maintien du cadre et/ou des mutations ponctuelles aboutissant à des variants protéiques fonctionnels, et excluant les mutations qui introduisent un codon d'arrêt prématuré entraînant une perte de fonction, de la séquence cible identifiée à l'étape (vi).

2. Procédé selon la revendication 1, dans lequel l'endonucléase guidée par ARN est choisie dans le groupe constitué par CRISPR/Cas9 Cpf1, Cas9-VQR, Cas9-EQR, Cas9-VRER, AsCpf1-S542R/K607R, AsCpf1-S542R/K548V/N552R, LbCpf1-G532R/K595R, LbCPf1-G532R/K538V/Y542R.

3. Procédé selon la revendication 1 ou 2, dans lequel la banque de vecteurs est de type « tiling ».

4. Procédé selon l'une quelconque des revendications 1 à 3, dans lequel l'étape de sélection (ii) et/ou l'étape (iii) est/sont basée(s) sur la sélection de clones survivants ou sur un autre phénotype pouvant être sélectionné ou enrichi.

5. Procédé selon l'une quelconque des revendications 1 à 4, dans lequel le stimulus est une molécule bioactive ayant une activité anticancéreuse ou une molécule bioactive induisant un phénotype pouvant être sélectionné ou enrichi.

6. Procédé selon l'une quelconque des revendications 1 à 5, dans lequel le stimulus est un médicament, un agent pathogène, un virus ou une bactérie.

7. Procédé selon l'une quelconque des revendications 1 à 6, dans lequel la banque de vecteurs est une banque de vecteurs lentiviraux, ou dans lequel la banque de vecteurs comprend tous les ARN guides possibles présents dans la séquence codante d'au moins un gène cible candidat ou présents dans l'exome entier de l'organisme que le stimulus cible.

8. Procédé selon l'une quelconque des revendications 1 à 7, dans lequel l'endonucléase guidée par ARN est fusionnée avec une enzyme de réparation d'ADN, ou dans lequel le guide ARN recrute une enzyme de réparation d'ADN telle qu'une β-polymérase, une θ-polymérase ou une ADN ligase IV, y compris tout mutant de celles-ci.

9. Procédé selon l'une quelconque des revendications 1 à 8, dans lequel la banque de vecteurs de l'étape (i) comprend un marqueur de sélection et dans lequel, dans l'étape (ii), les cellules transduites sont sélectionnées en utilisant ce marqueur.

10. Procédé selon la revendication 9, dans lequel le marqueur est un marqueur de résistance à un antibiotique, et les cellules transduites à l'étape (ii) sont sélectionnées par croissance des cellules en présence dudit antibiotique.

11. Procédé selon l'une quelconque des revendications 1 à 10, dans lequel le stimulus est létal pour la cellule de type sauvage non traitée et dans lequel le stimulus est létal pour toutes les cellules dans l'étape (iii) qui ne comprennent pas une mutation conférant une résistance audit stimulus à la fin de l'étape (ii).

12. Procédé selon l'une quelconque des revendications 1 à 11, dans lequel l'étape (i) est d'environ 1 jour et/ou l'étape (ii) est d'environ 5 jours et/ou l'étape (iii) est d'environ 1 à 2 semaines et/ou l'étape (iv) est d'environ 1 à 2 semaines.

13. Procédé selon l'une quelconque des revendications 1 à 12, dans lequel le stimulus agit sur un gène essentiel et la séquence cible à l'étape (vi) fait partie dudit gène essentiel.

14. Procédé de génération d'une lignée cellulaire mutante, comprenant:
(i) transduire une lignée cellulaire, exprimant de manière stable une endonucléase guidée par ARN du système de répétitions palindromiques courtes espacées régulièrement et groupées (CRISPR), ladite endonucléase générant des mutations par jonction d'extrémités non homologues (NHEJ), avec une banque de vecteurs comprenant des ARN guides ciblant des séquences exoniques, et ciblant des séquences introniques à l'intérieur de 30 paires de bases d'une limite intron-exon, dans au moins un gène cible candidat ou de l'exome entier de l'organisme visé par le stimulus,
étant entendu que le procédé ne comprend pas l'utilisation d'un modèle ou d'un substrat de réparation dirigée par homologie (HDR) ;
(ii) sélectionner les cellules transduites à la fin de l'étape (i) ;
(iii) sélectionner, à la fin de l'étape (ii), les cellules ayant un certain phénotype ;
(iv) développer les colonies sélectionnées à la fin de l'étape (iii) ;
(v) identifier ou séquencer la ou les séquence(s) d'ARN guides présentes dans les colonies sélectionnées générées en (iv) ;
(vi) séquencer la région génomique autour de la séquence cible du ou des ARN guide(s) identifié(s) pour identifier les mutations qui provoquent ledit phénotype particulier ; et
(vii) sélectionner les colonies dans lesquelles les mutations sont des insertions avec maintien du cadre et/ou des délétions avec maintien du cadre et/ou des mutations ponctuelles de la séquence cible identifiée à l'étape (vi).

15. Procédé selon la revendication 14, dans lequel l'endonucléase guidée par ARN est choisie dans le groupe constitué par CRISPR/Cas9 Cpf1, Cas9-VQR, Cas9-EQR, Cas9-VRER, AsCpf1-S542R/K607R, AsCpf1-S542R/K548V/N552R, LbCpf1-G532R/K595R et LbCPf1-G532R/K538V/Y542R.
